# EUROPÄISCHE PATENTSCHRIFT

(11) **EP 2 200 585 B1**
(45) Veröffentlichungstag und Bekanntmachung des Hinweises auf die Patenterteilung: **26.03.2014**
(21) Anmeldenummer: 08784291.0
(22) Anmeldetag: 03.07.2008
(51) Int. Cl.: A61K 45/06, A61K 9/06, A61K 47/32, A61K 47/36, A61K 9/70, A61K 33/00, A61P 31/22, A61K 9/00, A61K 9/12

(54) **VERWENDUNG VON DEUTERIUMOXID ZUR BEHANDLUNG VON VIRUS-BASIERTEN ERKRANKUNGEN DER HAUT**
USE OF DEUTERIUM OXIDE FOR TREATING VIRUS-BASED SKIN DISEASES
UTILISATION D'OXYDE DE DEUTERIUM POUR TRAITER DES AFFECTIONS CUTANEES D'ORIGINE VIRALE

(30) Priorität: 05.07.2007 DE 102007031397
(43) Veröffentlichungstag der Anmeldung: 30.06.2010
(60) Teilanmeldung: 12154192.4
(73) Patentinhaber: D2 Bioscience Group Ltd, Hamilton HMLX (BM)
(72) Erfinder: BAYERL, Thomas, London SW1X 0DE (GB)
(74) Vertreter: Ettmayr, Andreas
(86) Internationale Anmeldenummer: PCT/DE2008/001103
(87) Internationale Veröffentlichungsnummer: WO 2009/003463

(56) Entgegenhaltungen:
- WO-A-99/62510
- WO-A-2005/016234
- WO-A-2008/046407
- DE-A1- 3 019 434
- DE-A1- 4 427 690
- US-A- 5 788 953
- MÜLLER-BREITKREUTZ K ET AL: "Inactivation of viruses by chemically and photochemically generated singlet molecular oxygen." JOURNAL OF PHOTOCHEMISTRY AND PHOTOBIOLOGY. B, BIOLOGY SEP 1995, Bd. 30, Nr. 1, September 1995 (1995-09), Seiten 63-70, XP002501205 ISSN: 1011-1344
- 'Deuterium', [Online] Gefunden im Internet: <URL:Wikipedia>

## Beschreibung

Die vorliegende Erfindung betrifft die Verwendung von Deuteriumdioxid (D20) zur Prophylaxe und/oder Therapie von Herpesvirus-basierten Erkrankungen der Haut.

Durch Viren hervorgerufene Erkrankungen (Virusinfektionen) sind weltweit verbreitet und stellen, insbesondere aufgrund der hohen Variabilität, Anpassungsfähigkeit und Mutationsrate von Viren, ein ernsthaftes Problem in der Medizin dar. Viren sind kleine Partikel von ca. 15 bis 400 nm, die nicht in der Lage sind, sich selbst zu replizieren, sondern hierfür eine Wirtszelle benötigen. Ihrer Wirtsspezifität entsprechend werden Viren unterschieden, die Tiere (Invertebraten und Vertebraten), Pflanzen, Bakterien oder Algen, Pilze und Protozoen befallen. Virusinfektionen zeichnen sich allgemein durch eine hohe Vermehrungsrate der Viruspartikel in den befallenen Wirtszellen aus, welche mit einem Exponential- oder Potzenzgesetz beschrieben werden kann. Der allgemeine Vermehrungszyklus von Viren erfolgt über die Injektion ihrer Nukleinsäure (RNA oder DNA) in die Wirtszelle, in welcher die Replikation der Nukleinsäure durch Ausnutzung des Replikationsapparates der Wirtszelle erfolgt. Die Vermehrung von Viren kann entweder im lytischen oder lysogenen Zyklus erfolgen. Bei dem lytischen Zyklus (aktive Infektion), erfolgt nach der Injektion der Nukleinsäure die Replikation der Virus-Nukleinsäure im Zellkern der Wirtszelle, ein Zusammenbau der neuen Viruspartikel im Cytoplasma, worauf folgend die Wirtszelle schließlich lysiert (zerstört) wird und die Viren freigesetzt werden. Die so freigesetzten Viren infizieren weitere Wirtszellen. Bei dem lysogenen Zyklus wird die Nukleinsäure (DNA) des Virus in das Genom der Wirtszelle integriert, wo sie zunächst verbleibt ohne die Wirtszelle zu zerstören. Durch äußere Einflüsse (z.B. UV-Strahlung, Zugabe von mutagenen Substanzen) kann dieser lysogene Zyklus in einen vorangehend beschriebenen lytischen Zyklus übergehen.

Es sind im Stand der Technik verschiedene Behandlungansätze gegen Vireninfektionen bekannt.

Müller-Breitkreutz et al. beschreibt die Inaktivierung von Viren, wie HSV-1 oder HIV-1, durch Bestrahlung in Gegenwart photoreaktiver Farbstoffe. Es wird vermutet, dass chemisch hergestellter Singulett-Sauerstoff (¹O₂) zu dem viruziden Effekt dieser Photosensibilisierung beiträgt ("Inactivation of viruses by chemically and photochemically generated singlet molecular oxygen." JOURNAL OF PHOTOCHEMISTRY AND PHOTOBIOLOGY. B, BIOLOGY SEP 1995, Bd. 30, Nr. 1, September 1995 (1995-09), Seiten 63-70, XP002501205 ISSN: 1011-1344).

W02005/016234 beschreibt Verfahren, Vorrichtungen und Systeme zur Verabreichung von Singulett-Sauerstoff in der Behandlung von Tumoren, Läsionen und Krebserkrankungen sowie zur Bekämpfung von Bakterien, Viren, Pilzen, ein- und mehrzelligen Organismen. Das Singulett-Sauerstoff herstellende System umfasst eine Peroxidquelle und eine Hypochloritanionenquelle und eine Isotopenquelle.

DE3019434 beschreibt die Entkeimung von lebenden Zellen mit einem Deuteriumangereichtertem Wasser. Dieses Wasser soll zum Baden oder Trinken verwendet werden und zum Abbau von Viren und Bakterien geeignet sein.

Unter Virus-basierten Erkrankungen der Haut werden allgemein Erkrankungen der Haut verstanden, die durch ein Virus hervorgerufen werden. Unter den Virus-basierten Erkrankungen der Haut sind insbesondere durch Herpesviren hervorgerufene Erkrankungen bzw. Infektionen bekannt, die im allgemeinen Sprachgebrauch auch einfach als Herpes bezeichnet werden. Herpesviren sind bei Wirbeltieren, insbesondere bei Säugetieren, und vor allem bei Mensch, Pferd, Schwein, Rind, Ziege, Schaf, Katze und Hund weit verbreitet.

Humanpathogene Herpesviren (HHV) werden in Alpha-, Beta- und Gamma-Herpesviren (HHV-1 bis HHV-8) unterschieden, wobei den Alpha- und Gammaviren auch Viren angehören, die Tiere infizieren können, wie beispielsweise Pferd (Equines Herpesvirus), Rind (bovines Herpesvirus), Schwein (porcines Herpesvirus), Katze (felines Herpesvirus), Hund (canines Herpesvirus) und Huhn (Hühner-Herpesvirus 1).

Unter den humanpathogenen, d.h. den Menschen befallenden, Herpesviren sind insbesondere die Alpha-Herpesviren von großer Bedeutung. Zu den Alpha-Herpesviren werden das Herpes simplex Virus 1 (HSV-1), das Herpes simplex Virus 2 (HSV-2) und das Varizella Zoster Virus (VZV) gezählt.

Zu den durch Herpes Simplex Viren HSV-1 und HSV-2 hervorgerufenen Hauterkrankungen gehören beispielsweise Herpes labiales, auch als Lippenherpes oder Fieberbläschen bezeichnet, und Herpes nasalis, beide überwiegend durch HSV-1 ausgelöst, Keratoconjunctivitis herpetica, Stomatitis herpetica, Herpes facialis, Herpes buccalis, Herpes genitales, Herpes perianalis, Herpes glutealis. Varizella Zoster Viren (VZV) rufen beispielsweise Windpocken und Gürtelrose hervor.

Virologen gehen davon aus, dass 80% der Bevölkerung weltweit mit HSV-1 und 30% mit HSV-2 latent infiziert sind. HSV-1 und HSV-2 sind eng miteinander verwandt und weisen einen großen Anteil identischer Nukleotidsequenzen auf. Während HSV-1 bevorzugt den Mundbereich und das Gesicht infiziert, findet man HSV-2 meistens im Genitalbereich.

Alpha-Herpesviren infizieren in der Regel zunächst Epithelzellen, wie Haut- Schleim- und Schleimhautzellen, gefolgt von einer starken Vermehrung des Virus in der Wirtszelle und einem Absterben der infizierten Wirtszelle.

Beispielsweise erfolgt die Ausbreitung von HSV-1 in Haut- bzw. Schleimhautzellen von Gesicht und Mund und ruft in der Regel eine lokale Schädigung hervor, und zwar in Form eines Bläschens. Antikörper neutralisieren die Viren in den Bläschen nach kurzer Zeit, jedoch werden auch Viren durch Lyse der infizierten Wirtszellen freigesetzt, bevor eine humorale Immunantwort stattfinden kann. Die freigesetzten Viren infizieren weiterhin bestimmte Nervenzellen (Neuronen), indem sie sich im Mund an Rezeptoren der Nervenenden anheften, die zu den Nervenknoten des Gesichtsnervs (Trigeminus) führen. Die Virus-DNA dringt in ein Axon ein, wird in das Cytoplasma der Nevenzellen und schließlich in deren Zellkern befördert. Dort erfolgt der Einbau der Virus-DNA in das Genom der Nervenzelle und führt zu einem Ruhezustand (Latenz), in dem nur wenige virale Gene exprimiert werden (lysogener Zyklus). Verschiedene äußere Reize können zu_einer erneuten Aktivierung des Virus führen, d.h. zu dem Übergang von der Latenz bzw. dem lysogenen Zyklus zum lytischenen Zyklus, welches letztendlich die Zerstörung (Lyse) der Nervenzelle zur Folge hat. Derartige Reize sind beispielsweise Immunsuppression, Stress, Hormonschwankungen, körperliche oder seelische Belastungen, Fieber (deshalb spricht man auch von Fieberbläschen), UV-Strahlung, Gewebeschäden und in manchen Fällen eine Immunschwäche. Die Häufigkeit einer solchen Aktivierung von HSV-1 ist sowohl von genetischen als auch von umweltbedingten Faktoren abhängig, und auch Alter und Hormonveränderungen spielen dabei eine Rolle. Die im Rahmen einer Aktivierung entstehenden Virusnachkommen werden zunächst durch das Axon an die Stelle bzw. in das Gebiet der ursprünglichen Hautinfektion transportiert und erzeugen dort durch Infektion von und Vermehrung in den Haut- bzw. Schleimhautzellen wiederum Bläschen (Fieberbläschen). Selbst wenn diese Viren wiederum vom Immunsystem neutralisiert werden, ist eine vollständige Befreiung des Körpers von HSV-1 durch das Immunsystem nicht möglich, da ständig weitere Nervenzellen infiziert werden, in denen die Virus-DNA integriert im Genom verbleibt (Latenz).

Weitere Virus-basierte Erkrankungen der Haut, die weit verbreitet sind, sind durch Warzenviren hervorgerufene Erkrankungen bzw. Infektionen, die im allgemeinen Sprachgebrauch als Warzen bezeichnet werden.

Warzen (Verrucae) sind, unter Umständen sehr ansteckende, kleine, scharf begrenzte, leicht erhabene oder flache, im allgemeinen gutartige Epithelgeschwülste bzw. Wucherungen der Haut, insbesondere der Epidermis. Sie treten insbesondere an Hand und Fuß auf. Warzen haben meistens einen dicken, hornigen, teilweise auch zerklüfteten Überzug aus Plattenepithelzellen unter dem sich weiches Gewebe aus Keratinocyten befindet.

Ausgelöst werden diese Hauterkrankungen (Warzen) durch eine Virusinfektion, vor allem durch Papillomaviren, insbesondere humane Papillomaviren (HPV), wie beispielsweise Verrucae vulgares, Verrucae planae juvenilis, Verrucae plantares, Condylomata acuminata, Verrucae planae, Verrucae filiformes und Molluscipicoviren, z. B. Molluscum contagiosum. Die Infektion erfolgt über kleine Verletzungen der Haut und der Schleimhäute. Warzenviren infizieren Hautzellen von Wirbeltieren, insbesondere Säugetieren, und vor allem vom Mensch.

Bei Warzen handelt es sich grundsätzlich um gutartige Wucherungen, die an bestimmten Stellen, z. B. im Genitalbereich, starke Schmerzen verursachen können und sich - extrem selten - maligne entwickeln können. Durch ihr Erscheinungsbild und eine häufig auftretende weitere Ausbreitung auf der Haut können sie weiterhin auch kosmetisch stören. Eine Behandlung von Warzen ist sehr langwierig. Bislang sind verschiedene Behandlungsansätze entwickelt worden:
- Chirugische Entfernung
- Elektrokoagulation
- Entfernung durch Laser
- Kyrochirugie (Vereisung)
- Kauterisation (Einsatz von elektrischem Strom oder Ätzmitteln)
- Zytostatika, wie 5-Fluoruracil, Podophyllotoxin und Podophyllin bekannt, mit denen die Warze eingestrichen wird.

Alle vorstehend erläuterten Behandlungsansätze basieren jedoch auf einer Behandlung der Folgen einer Warzenvirusinfektion, nicht auf eine Hemmung oder Verhinderung der Virusvermehrung.

Im Gegensatz dazu erfolgt die Behandlung der erwähnten Herpesvirus-basierten Erkrankungen der Haut allgemein durch den Einsatz von antiviralen Wirkstoffen, die insbesondere in die Virusvermehrung eingreifen. Hierauf wird nachfolgend näher eingegangen.

Ein wichtiger allgemeiner Ansatz bei der Entwicklung von antiviralen Wirkstoffen, und somit auch bei der Prophylaxe und/oder Therapie von Virus-basierten Erkrankungen der Haut, ist es demnach, in den Vermehrungszyklus des Virus einzugreifen und bereits die virale Infektion der Wirtszelle oder die Vermehrung der viralen Nukleinsäure in der Wirtszelle zu inhibieren (d.h. verhindern) oder zu hemmen, beispielsweise indem die Expression der durch die virale Nukleinsäure kodierten viralen Proteine gehemmt oder inhibiert wird.

In den letzten Jahrzehnten sind solche antiviralen Wirkstoffe, sogenannte Virostatika, entwickelt worden. Viele Virostatika hemmen beispielsweise die Enzyme DNA-Polymerase, reverse Transkriptase oder Proteasen und hemmen oder inhibieren somit die Replikation des Virus bzw. die Prozessierung eines synthetisierten langen Virusproteins in kleinere Proteinabschnitte. Beispiele für diese therapeutischen Ansätze finden sich besonders in der Therapie von HIV Infektionen. Aber auch im Bereich der Therapie von Virus-basierten Hauerkrankungen, die durch die Herpesviren HSV-1 oder HSV-2 ausgelöst werden, sind Virostatika bekannt, welche systemisch oder topisch verabreicht werden. Beispiele hierfür sind die Wirkstoffe Aciclovir, Valaciclovir, Foscarnet und Peniclovir.

Zur Prophylaxe und/oder Therapie von Virus-basierten Erkrankungen der Haut mit Virostatika sind generell zwei Ansätze bekannt:
(1) die systemische Verabreichung: durch systemische Verabreichung von Virostatika kann eine signifikante Verringerung der Aktivierung von in Wirtszellen vorhandenen Viren erreicht werden, da die Wirkstoffe die Vermehrung der Virus-Nukleinsäure im Zellkern oder den Zusammenbau der Viruspartikel zu vollständigen Viren im Cytoplasma der Wirtszellen hemmen oder inhibieren;
(2) die topische Verabreichung: durch topische Applikation von Virostatika in dem Gebiet einer Erstinfektion durch das Virus kann in einem frühen Stadium der Aktivierung der weitere Weg der Vermehrung der Viren, beispielsweise in den sich bildenden Fieberbläschen bei einer HSV-1-Infektion, behindert werden, welches zu einem schnelleren Abschwellen der Fieberbläschen führen kann.

Beide Ansätze für die Verabreichung von Virostatika haben bedeutsame Nachteile:
- bei einer systemischen Verabreichung ist die für eine effektive Behandlung erforderliche Dosis relativ hoch verbunden mit starken Nebenwirkungen für den behandelten Organisums, wie beispielsweise unspezifische Immunantworten und Autoimmunreaktionen. Für Fall von Aciclovir sind zahlreiche solche Nebenwirkungen aus der Literatur bekannt. Weder eine Langzeit-Therapie noch Wiederholungs-Therapien sind daher ratsam und einem Patienten zuzumuten;
- bei der topischen Applikation ist die Menge an Wirkstoff (Virostatikum), welche pro Zeiteinheit in dem Gebiet der Virusinfektion, beispielsweise des Fieberbläschens, freigesetzt und bioverfügbar werden kann, sehr gering. Diese geringe Bioverfügbarkeit des Virostatikums stellt ein wesentliches Hindernis für eine effektive topische Therapie dar. Für den Fall des nur schwer wasserlöslichen Aciclovir liegt die geringe Bioverfügbarkeit beispielsweise an dem schlechten perkutanen Transport des Wirkstoffs. Auch verschiedene chemische Modifikationen von Virostatika im Rahmen von Pro-Drug Konzepten für eine verbesserte Wirkstoffzufuhr von Virostatika haben zu keiner Verbesserung dieses Phänomens geführt.

Unter den in der Literatur beschriebenen Virostatika zur Behandlung von Virus-basierten Erkrankungen der Haut wird das bereits genannte Aciclovir als eines der effektivsten Virostatika beschrieben wird, zu welchem nahezu keine Alternativen existieren.

Alternativen zu bekannten antiviralen Wirkstoffen, insbesondere den Virostatika, zur Prophylaxe und/oder Therapie von Virus-basierten Erkrankungen der Haut, welche die im Stand der Technik bekannten Nachteile überwinden, gibt es nicht. Es besteht daher Bedarf, verbesserte und verträglichere antivirale Wirkstoffe zu entwickeln, die in den Vermehrungszyklus des Virus einzugreifen und vorzugsweise bereits die virale Infektion der Wirtszelle und die Vermehrung der viralen Nukleinsäure in der Wirtszelle inhibieren (d.h. verhindern) oder zu hemmen.

Aufgabe der Erfindung ist es demnach, verbesserte antivirale Wirkstoffe zur Prophylaxe und/oder Therapie von Virus-basierten Erkrankungen der Haut bereitzustellen.

Diese Aufgabe wird durch die vorliegende Erfindung gelöst. Die Erfindung betrifft die Verwendung von Deuteriumoxid zur Prophylaxe und/oder Therapie von Herpesvirus-basierten Erkrankungen der Haut sowie die Verwendung von Deuteriumoxid zur Herstellung eines Arzneimittels zur Prophylaxe und/oder Therapie von Herpesirus-basierten Erkrankungen der Haut.

Die Erfindung betrifft in ihrem ersten Gegenstand Deuteriumoxid zur Verwendung für die Prophylaxe und/oder Therapie von Herpesvirus-basierten Erkrankungen der Haut, wobei Deuteriumoxid rein, oder in Lösung mit Wasser als Lösungsmittel in einer Konzentration von 1 % bis 98% bezogen auf den Gesamtwassergehalt der Lösung, vorliegt.

Bevorzugt werden die Herpesvirus-basierten Erkrankungen der Haut durch Herpes simplex Virus Typ 1 oder Herpes simplex Virus Typ 2 hervorgerufen. Besonders bevorzugt handelt es sich um Herpesvirus-basierte Erkrankungen der Haut, ausgewählt aus der Gruppe bestehend aus Herpes labiales, Herpes nasalis, Keratoconjunctivitis herpetica, Stomatitis herpetica, Herpes facialis, Herpes buccalis, Herpes genitales, Herpes perianalis und Herpes glutealis und Herpes zoster.

Unter "Virus-basierten Erkrankungen der Haut" werden Erkrankungen der Haut verstanden, die durch ein Virus hervorgerufen werden. Die Begriffe "Virus-basierte Erkrankung", "Viruserkrankung" und "Virusinfektion" der Haut werden im Sinne der Erfindung synonym angewendet. Unter "Herpesvirus-basierte Erkrankungen der Haut" werden Erkrankungen der Haut verstanden, die durch ein Herpesvirus hervorgerufen werden.

Die Begriffe "Prophylaxe und/oder Therapie" bezeichnen erfindungsgemäß jede für die Behandlung einer Virus-basierten Erkrankung der Haut geeignete Maßnahme, die entweder eine vorbeugende Behandlung einer solchen sich abzeichnenden Erkrankung bzw. deren sich abzeichnende Symptome darstellt (Prophylaxe) oder die Vermeidung eines Wiederausbrechens einer solchen Erkrankung, beispielsweise nach einem abgeschlossenen Behandlungszeitraum, oder die Behandlung der Symptome einer solchen bereits ausgebrochenen Erkrankung darstellt (Therapie).

Die wirksame Prophylaxe oder Therapie einer Virus-basierten Erkrankung der Haut kann insbesondere durch Verabreichung eines pharmazeutischen Wirkstoffes bzw. eines antiviralen Wirkstoffes erfolgen, der unter Berücksichtigung der Art seiner Verabreichung (z.B. systemisch oder topisch), vorzugsweise alle der nachstehenden Eigenschaften aufweist:
a) bei topisch zu verabreichenden antiviralen Wirkstoffen: eine lokale Anwendbarkeit durch topische Verabreichung über einen beliebig langen Zeitraum und mit hoher perkutaner Transportkinetik des Wirkstoffs;
b) eine weitgehend homogene Wirkstoffverteilung im Bereich des Wirkortes sowie die Vermeidung von lokalen Überkonzentrationen;
c) eine bevorzugte Anreicherung des antiviralen Wirkstoffes in den vom Virus befallenen Hautgebieten, möglichst verbunden mit einer Retardierung seines transdermalen Transportes in den Blutkreislauf, d.h. in die Blutgefäße;
d) eine virustatische Wirkung, d.h. eine die Vermehrung von Viren hemmende Wirkung, auf die (Wirts-)Zellen im betroffenen Hautgebiet. D.h. die Virusvermehrung durch den wirtszelleigenen Replikationsapparat wird gehemmt, vorzugsweise inhibiert;
e) weitgehende Toleranz des antiviralen Wirkstoffs durch die gesunden (vom Virus nicht infizierten) Zellen im Hautgewebe sowie den Blutkreislauf zur Vermeidung von Nebenwirkungen, insbesondere auch im Hinblick auf Immunreaktionen.

Erfindungsgemäß wurde festgestellt, dass Deuteriumoxid (D20) diese vorgenannten Anforderungen erfüllt und darüber hinaus gegenüber den im Stand der Technik bekannten antiviralen Wirkstoffen, insbesondere den Virostatika, deutliche Vorteile aufweist, beispielsweise weitaus geringere bzw. keine Nebenwirkungen sowie erhöhte Bioverfügbarkeit. Erfindungsgemäß wurde weiterhin festgestellt, dass Deuteriumoxid ist ein effektiver antiviraler Wirkstoff mit potentieller Langzeitwirkung ist, der sowohl für Kurz-, Langzeit- und Wiederholungs-Therapien von Virus-basierten Erkrankungen der Haut geeignet ist. Erfindungsgemäß wird Deuteriumoxid, nachfolgend D20 genannt, auch als antiviraler Wirkstoff bezeichnet.

Bevor auf Deuteriumoxid als erfindungsgemäßer antiviraler Wirkstoff näher eingegangen wird, werden nachfolgende erläuternde Definitionen gegeben.

Unter dem Begriff "pharmazeutischer Wirkstoff" wird erfindungsgemäß jede(s) beliebige anorganische oder organische Molekül, Substanz oder Verbindung, das/die eine pharmakologische Wirkung aufweist verstanden. Der Begriff "pharmazeutischer Wirkstoff" wird hierin mit dem Begriff "Arzneimittel" synonym verwendet. Zu den pharmazeutischen Wirkstoffen im Sinne der Erfindung gehören auch antivirale Wirkstoffe einschließlich D20.

Der Begriff "antiviraler Wirkstoff" im Sinne der Erfindung definiert einen Wirkstoff, der zur Behandlung von Virus-basierten Erkrankungen, und insbesondere Virus-basierten Erkrankungen der Haut, einsetzbar ist. Vorzugsweise ist ein solcher antiviraler Wirkstoff geeignet, die virale Infektion der Wirtszelle und/oder die Vermehrung der viralen Nukleinsäure in der Wirtszelle zu inhibieren oder zu hemmen. Sofern ein antiviraler Wirkstoff die Virusvermehrung hemmt oder inhibiert, wird auch der Begriff "virustatischer Wirkstoff" verwendet.

"Zellen" im Sinne der Erfindung sind tierische Zellen, bevorzugt humane Zellen, und zwar Hautzellen, insbesondere, aber nicht hierauf beschränkt, Epithelzellen der Haut, wie Schleim- und Schleimhautzellen. Bei "Wirtszellen" handelt es sich erfindungsgemäß um solche Hautzellen, die von einem Virus infiziert sind oder infiziert werden können.

Die Eigenschaften von D20 sowie dessen Wirkungsmechanismus als erfindungsgemäßer antiviraler Wirkstoff stellen sich wie folgt dar:
Deuteriumoxid (D20), häufig auch als "schweres Wasser" bezeichnet, ist eine dem "natürlichen Wasser" H2O in seinen physikalischen Eigenschaften äußerst ähnliche Substanz. Deuteriumoxid (D20) und Wasser (H2O) unterscheiden sich physikalisch durch die Substitution der Wasserstoffatome von H2O durch Deuteriumatome, wobei D20 eine ca. 10% höhere Dichte und eine ca. 25% höhere Viskosität aufweist. Darüber hinaus sind Schmelz- und Siedetemperaturen von D20 höher als für H2O. Ein detaillierter Vergleich der Eigenschaften wird im Handbook of Chemistry and Physics, Sektion 6, gegeben (Handbook of Chemsitry and Physics, David R. Lide, Editor, 79. Auflage, 1998 CRC Press, Boca Raton, USA) dargestellt.

Neben den physikalischen ähnlichen Eigenschaften von H2O und D2O bestehen bedeutsame physiologische Unterschiede (siehe u.a. Kushner DJ et al., Pharmacological uses and perspectives of heavy water and denatured compounds., Can J Physiol Pharmacol. 1999 Feb;77(2):79-88.). D20 weist ab einer gewissen Konzentration in einer Zelle, bei tierischen Zellen von mehr als 20 - 25 %, eine Wirkung auf enzymatische Reaktionen auf. Enzymatisch gesteuerte Reaktionen werden zunehmend verändert, insbesondere gehemmt oder inhibiert. Eine Ursache hierfür wird in der höheren Bindungsstärke der Wasserstoffbrückenbindungen, wenn das Wasserstoffatom von H2O durch ein Deuteriumatom substituiert ist, gesehen. Sowohl in wässrigen Lösungen von H2O und D2O, als auch bei der Bindung von Wasser an organische Moleküle, tritt diese erhöhte Bindungsstärke auf (Cuma M, Scheiner S, Influence of Isotopic Substitution on Strength of Hydrogen Bonds of Common Organic Groups, Journal of Physical Organic Chemistry, 1997 Band 10, 383-395).

Es ist allgemeines Fachwissen, dass eine Zelle abhängig von dem Grad ihrer jeweiligen metabolischen Aktivität unterschiedliche Mengen H2O aufnimmt. Eine Zelle, die von einem Virus infiziert wird, sog. Wirtszelle, und in der damit eine Vermehrung des Virus stattfindet, weist eine weitaus höhere metabolische Aktivität auf als eine nicht infizierte Zelle desselben Zelltyps des umliegenden Gebiets. Der Grund hierfür ist, dass die Wirtszelle nicht nur ihre eigene Replikation, sondern auch die Replikation des Virus bewirkt. Da eine erhöhte metabolische Aktivität von Zellen mit einer erhöhten Wasseraufnahme korreliert, nehmen Virus-infizierte Wirtszellen deutlich mehr Wasser (H2O) auf als nicht infizierte Zellen. Aufgrund der ähnlichen physikalischen Eigenschaften von H2O und D20, wird D20 von Zellen parallel zu H2O (wenn D20 und H2O verfügbar sind) oder anstelle von H2O (wenn nur D20 verfügbar ist) aufgenommen.

Wie vorstehend erläutert, werden enzymatische Reaktionen in einer Zelle durch D20 in ausreichender Konzentration verändert. Wie ebenfalls vorstehend erläutert, nehmen Zellen, und Virus-infizierte Zellen in erhöhtem Ausmaß, D20 parallel oder anstelle von H2O auf. Wird demnach an eine Virus-infizierte Zelle D20 in einer erfindungsgemäß "ausreichenden Konzentration", d.h. mehr als 20% bezogen auf den Gesamtwassergehalt einer Zelle, verabreicht, hat dies eine Hemmung bzw. Inhibierung enzymatischer Reaktionen in der Wirtszelle zur Folge. Hierzu gehört insbesondere die Hemmung oder Inhibierung der DNA-Polymerase (Takeda H et al. Mechanisms of cytotoxic effects of heavy water (deuterium oxide: D20) on cancer cells, Anticancer Drugs. 1998 Sep;9(8):715-25). Als Folge hiervon wird die DNA-Replikation in einer Zelle gehemmt bzw. inhibiert. Nicht infizierte Zellen in dem umgebenden Gebiet nehmen D20 bzw. H2O in normalem Maß auf, so dass in diesen keine Hemmung oder Inhibierung enzymatischer Reaktionen erfolgt.

Erfindungsgemäß ist nunmehr festgestellt worden, dass, wenn eine Zelle mit einem Virus infiziert (Wirtszelle), durch die Hemmung bzw. Inhibierung der wirtszelleigenen DNA-Polymerase auch die Virus-DNA nicht repliziert wird, da deren Replikation ebenfalls durch die Wirtszell-DNA-Polymerase erfolgt. Im Fall von RNA-Viren erfolgt die enzymatische Hemmung bzw. Inhibierung durch D20 auch für Synthese der reversen Transkriptase, die zunächst, durch Virusgene kodiert, in der Wirtszelle synthetisiert werden muss, um die Virus-RNA in DNA umzuschreiben, die dann wiederum von der DNA-Polymerase der Wirtszelle repliziert wird. Eine Hemmung bzw. Inhibierung bestimmter enzymatischer Reaktionen der Wirtszelle durch D20 hemmt bzw. verhindert demnach auch die Virusvermehrung.

Ein weiterer wichtiger Aspekt der Erfindung, der auf der beschriebenen erhöhten Bindungseigenschaft von D20 basiert, ist, dass bei Verabreichung von D20 in ausreichender Konzentration, d.h. mehr als 20%, in einer Zelle, die Zellteilung gehemmt oder inhibiert wird. Dies geschieht höchstwahrscheinlich, zusätzlich zu der erläuterten Hemmung bzw. Inhibierung der DNA-Replikation, durch die Hemmung bzw. Inhibierung der Mitose im Zyklus der Teilung tierischer Zellen (Laissue JA et al. Survival of tumor-bearing mice exposed to heavy water or heavy water plus methotrexate, Cancer Research, 1982, Vol 42, (3) 1125-1129). Dies ist erfindungsgemäß von entscheidender Bedeutung für einen Latenzzustand bei Virus-basierte Erkrankungen der Haut, bei dem Viren im oben bereits beschriebenen Ruhezustand (Latenz) während des lysogenen Vermehrungszyklus vorliegen. In diesem Zustand wird das Virusgenom in das Wirtszellgenom integriert und bei Teilung der Wirtszelle zusammen mit dem Wirtsgenom auf die Wirtszellnachkommen übertragen. Bei einer Hemmung bzw. Inhibierung der Zellteilung wird so eine Übertragung des Virus auf neue Zellen verhindert.

Erfindungsgemäß erfolgt demnach eine Hemmung oder Inhibierung der Virusvermehrung durch die Verwendung von D20 zur Prophylaxe und/oder Therapie von Virus-basierten Erkrankungen der Haut. Diese Hemmung oder Inhibierung der Virusvermehrung durch D20 als antiviraler Wirkstoff erfolgt erfindungsgemäß insbesondere durch die Hemmung bzw. Inhibierung der Replikation der Virus-Nukleinsäure und/oder der Wirtszellteilung.

Unter dem Begriff "hemmen" bzw. "Hemmung" gemäß der Erfindung ist zu verstehen, dass die Replikation von Virus-Nukleinsäuren bzw. die Zellteilungsrate von Wirtszellen der Erfindung verlangsamt und/oder herabgesetzt wird, bevorzugt bis zu ca. 5%, vorzugsweise bis zu ca. 10%, ebenfalls bevorzugt bis zu ca. 20%, stärker bevorzugt bis zu ca. 30%, ebenfalls stärker bevorzugt bis zu ca. 40%, noch stärker bevorzugt bis zu ca. 50%, am stärksten bevorzugt bis zu ca. 60% gegenüber der Replikations- bzw. Teilungsrate dieser Zellen oder der viralen Vermehrungsrate ohne Verabreichung von D20.

Der Begriff "inhibieren" oder "Inhibierung" gemäß der Erfindung bedeutet, dass die Replikation von Virus-Nukleinsäuren bzw. die Zellteilungsrate von Wirtszellen der Erfindung, bevorzugt über 50%, ebenfalls bevorzugt bis zu ca. 60%, weiterhin bevorzugt bis zu ca. 65%, vorzugsweise bis zu ca. 70%, ebenfalls bevorzugt bis zu ca. 80%, stärker bevorzugt bis zu ca. 90%, ebenfalls stärker bevorzugt bis zu ca. 95%, noch stärker bevorzugt bis zu ca. 98%, am stärksten bevorzugt um bis zu 100% gegenüber der Replikations- bzw. Teilungsrate dieser Zellen oder der viralen Vermehrungsrate ohne Verabreichung des D20 verlangsamt bzw. herabgesetzt wird.

Eine solche antivirale bzw. virustatische Wirkung von D20 ist bislang im Stand der Technik nicht beschrieben oder angedeutet worden.

D20 weist gegenüber bekannten aniviralen bzw. virustatischen Wirkstoffen zur Behandlung von Virus-basierten Erkrankungen der Haut erhebliche Vorteile auf, vor allem durch folgende Eigenschaften:
1) durch die Möglichkeit, D20 topisch auf die Haut zu applizieren, kann wegen des hohen kutanen, perkutanen und intrakutanen Transports von D20 in die Hautzellen eine zur therapeutischen Wirksamkeit ausreichend hohe Konzentration (von mehr als 20% bezogen auf den Gesamtwassergehalt einer Zelle) von D20 in der Epidermis bzw. Dermis der Haut erreicht werden, ohne dass andere Organe des Körpers ähnlich hohen und möglicherweise schädlichen Konzentrationen von D20 ausgesetzt werden, wie es bei der systemischen Verabreichung erfolgen kann. Damit ist ein entscheidendes, im Stand der Technik diskutiertes, Problem zur Erreichung von therapeutisch wirksamen D2O-Konzentrationen am Wirkort (mehr als 20% D20 bezogen auf den Gesamtwassergehalt der Zelle) ohne starke Nebenwirkungen für andere Organe oder gesundes umliegendes Hautgewebe gelöst. Grundlage hierfür ist der gerichtete Transport von D20 durch das Stratum Corneum der Haut bis zur Epidermis bzw Dermis;
2) der Aggregatzustand von D20 kann bei einer topischen Verabreichung flüssig, gasförmig oder fest sein. Der Transport in die Haut kann durch direkten Kontakt der D20 bzw. einer D2O-enthaltenden Formulierung mit der Haut und indirekt durch Diffusion über eine zwischengelagerte Schicht (z.B. Luft, poröse Membran, polymeres Netzwerk) erfolgen;
3) in dem Fall, dass reines flüssiges D20 allein verabreicht wird (reines D20), ist auszuführen, dass D20 gegenüber allen anderen flüssigen pharmazeutischen Wirkstoffen einen einzigartigen Vorteil hat. Es kann wie normales Wasser (H2O) in die Haut transportiert werden und durch die Stärke und Richtung des osmotischen Gradienten und einer Manipulation dieser beiden Größen kann weiterhin die Eindringtiefe von D20 in die Haut an die therapeutische Zielsetzung angepasst werden;
4) die Wasserstoffbrückenbindungsstärke von D20 ist, wie bereits oben beschrieben, höher als bei H2O, insbesondere bei der Bindung von Wasser an organische Moleküle. Topisch verabreichtes D20 bindet molekular durch Wasserstoffbrückenbindungen an die nächste verfügbare Zelloberfläche und verdrängt dabei das dort angelagerte H2O aufgrund seiner höheren Bindungsstärke. Die Austausch-Frequenz der D20-Moleküle mit der H2O-Umgebung ist wiederum durch diese erhöhte Bindungsstärke (und durch das höhere Gewicht des D20-Moleküls) etwas langsamer als für H2O (König, S.,et al. "Molecular dynamics of water in oriented multilayers studied by quasi-elastic neutron scattering and deuterium-NMR relaxation".1994. J.Chem. Phys. 100, 3307-3316). Damit ergibt sich eine erhöhte Aufenthaltswahrscheinlichkeit der D20-Moleküle unmittelbar auf der Zelloberfläche, verbunden mit einer verstärkten Internalisierung von D2O in der Zelle, wodurch es seine Wirkung entfalten kann - die Hemmung oder Inhibierung der oben beschriebenen enzymatischen Reaktionen einer Virus-infizierten Wirtszelle sowie deren Teilung. Da Virus-infizierte Hautzellen eine höhere Aufnahmefähigkeit für Wasser bzw. D20 aufweisen als normale Zellen, ist darüber hinaus gesichert, dass D20 überproportional in diesen Zellen im Vergleich zu gesunden Hautzellen angereichert wird, d.h. in einer ausreichenden D2O-Konzentration von mehr als 20% bezogen auf den Gesamtwassergehalt der Zelle. Aufgrund dieser Eigenschaften ist die topische Applikation von D20 auf der Haut außerordentlich wertvoll für die Therapie von Virus-basierten Erkrankungen der Haut, bei denen der gewünschte Wirkort in der Epidermis oder höchstens in der Dermis der Haut liegt;
5) D20 ist das einzige nicht-radioaktive Molekül, welches dem H2O in seinen Eigenschaften sehr ähnlich ist. Zellen allgemein, und insbesondere Hautzellen, können zwischen den beiden Molekülen nicht "unterscheiden", so dass D20 durch aktiven und passiven Transport auf die gleiche Weise wie H2O in die Zelle transportiert wird und bis in die Zellkerne gelangt. Hierdurch werden Zellbarrieren beliebiger Arten, die ein Eindringen anderer pharmazeutischer Wirkstoffe verhindern, umgangen und ebenfalls Abwehrmechanismen auf zellulärer Ebene, wie die Internalisierung in Lysosomen oder die Aktivierung von MDR (multiple drug resistance) Transportern oder auf Organebene durch das Immunsystem, welche die Wirksamkeit des pharmazeutischen Wirkstoffs D20 reduzieren oder inhibieren könnten, sind weitgehend ausgeschaltet;
6) ein weiterer Vorteil von D20 als antiviraler bzw. virustatischer Wirkstoff ist die Tatsache, dass Konzentrationen unter 20% D20 (bezogen auf den Gesamtwassergehalt der Zelle) in der Zelle keine signifikanten Wirkungen entfalten und damit normale Zellen, die wegen ihrer gegenüber den aktiven, Virus-infizierten Zellen geringeren Wasserpermeabilität und/oder Wasseraufnahme vergleichsweise wenig D20 aufnehmen, kaum den Wirkungen des D20 ausgesetzt sind.

In einer besonders bevorzugten Ausführungsform der vorliegenden Erfindung wird D20 erfindungsgemäß nicht-systemisch, insbesondere topisch, verabreicht. Hierdurch werden vor allem Nebenwirkungen, die durch eine systemische Verabreichung von antiviralen bzw. virustatischen Wirkstoffen verursacht werden, vermieden.

Durch eine erfindungsgemäße topische Verabreichung können lokal hohe, therapeutisch wirksame D2O-Konzentrationen auf der Haut eingesetzt werden und gleichzeitig die Belastungen des Systems (d.h. des Blutkreislaufs) und die Nebenwirkungen auf nicht zu behandelndes, gesundes Hautgewebe sowie Gewebe von anderen Organen (wie beispielsweise der Leber oder Niere, die durch eine hohe Konzentration von D20 von mehr als 20% D20, bezogen auf den Gesamtwassergehalt der Zelle, verursacht werden könnten), zu vermindern bzw. vollständig zu vermeiden. Ein Transport von D20 von den Hautzellen in das System kann darüber hinaus mit Mitteln, die im Stand der Technik gut bekannt sind, verhindert oder eingeschränkt werden kann. Beispiele für diese Mittel sind u.a. die gezielte Manipulation des osmotischen Gradienten über die Haut (d.h. zwischen systemischen Teil und der Hautoberfläche) durch Verringerung des Wasserpotentials des topisch applizierten D20 mittels Substanzen, die geeignet sind, dieses Wasserpotential zu verändern, insbesondere physiologisch verträgliche Salze, wie Natriumchlorid, wasserlösliche Polymere und andere nicht-pharmazeutische Stoffe.

Der Begriff "topisch" bzw. "topische Applikation" oder "topische Verabreichung" oder "topische Anwendung" im Sinne der vorliegenden Erfindung bedeutet das lokale Auftragen oder Einbringen von D20 und weitern erfindungsgemäßen Wirkstoffen, z.B. pharmazeutischen Wirkstoffen oder nicht-pharmazeutischen Wirkstoffen, auf die Haut, bevorzugt als Flüssigkeit, Gas oder Formulierung, insbesondere Salbe, Creme, Gel oder Hydrogel.

Eine erfindungsgemäße topische Verabreichung erfolgt bevorzugt durch
1) Verabreichung als Flüssigkeit oder Formulierung, insbesondere Salbe, Creme oder Gel (nachfolgend näher beschrieben);
2) Verabreichung über ein Pflaster oder eine Bandage (nachfolgend näher beschrieben) oder
3) Verabreichung als Aerosol oder Dampf (nachfolgend näher beschrieben).

Ein zu behandelnder Organismus im Sinne der vorliegenden Erfindung ist ein tierischer Organismus, vor allem ein Wirbeltier, insbesondere ein Säugetier, vor allem ein Mensch, Pferd, Schwein, Rind, Ziege, Schaf, Katze und Hund.

D20 kann erfindungsgemäß allein als pharmazeutischer Wirkstoff, genauer als antiviraler bzw. virustatischer Wirkstoff, oder in Kombination mit einem weiteren pharmazeutischen Wirkstoff oder mit einem weiteren nicht-pharmazeutischen Wirkstoff (insbesondere zur Optimierung der topischen Applikation von D20 als pharmazeutischen Wirkstoff auf der Haut) verabreicht werden.

Eine bevorzugte Ausführungsform der vorliegenden Erfindung betrifft die erfindungsgemäße Verwendung von D20, wobei das D20 zusammen mit mindestens einem weiteren pharmazeutischen Wirkstoff und/oder mindestens einem weiteren nicht-pharmazeutischen Wirkstoff verwendet wird.

Eine solche Kombination aus D20 und mindestens einem weiteren pharmazeutischen Wirkstoff und/oder mindestens einem weiteren nicht-pharmazeutischen Wirkstoff wird nachfolgend als "erfindungsgemäße Kombination" bezeichnet.

Sämtliche in dieser Beschreibung offenbarten erfindungsgemäßen Verwendungen und Verabreichungen von D20, beispielsweise mit Pflaster und Bandagen, mit Schichtsystemen, in Formulierungen und als Aerosol, sind ebenfalls auf eine erfindungsgemäße Kombination uneingeschränkt anwendbar, sofern nichts Gegenteiliges angezeigt ist. Gleiches gilt für die Verwendung und Verabreichung von D20 in Kombination mit H2O, auch nachfolgend als "Mischung aus D20 und H2O" bezeichnet.

Eine weiterhin bevorzugte Ausführungsform der vorliegenden Erfindung betrifft die Verwendung von D20 zusammen mit mindestens einem weiteren pharmazeutischen Wirkstoff, wobei dieser ausgewählt ist aus der Gruppe, bestehend aus Virostatika, Proteinen, Peptiden, Nukleinsäuren und immunosuppressiven Wirkstoffen.

Nachfolgend werden erfindungsgemäß bevorzugte weitere pharmazeutische Wirkstoffe einer erfindungsgemäßen Kombination und deren Wirkung aufgeführt, wobei diese Aufzählung nur beispielhaft ist und die vorliegende Erfindung nicht hierauf beschränkt ist:
- Virostatika
   Als Virostatika werden Wirkstoffe bezeichnet, die die Vermehrung von Viren hemmen. Zahlreiche Virostatika hemmen die Aktivität von Enzymen, beispielsweise DNA-Polymerase, reverse Transkriptase oder Proteasen, und hemmen oder inhibieren somit die Replikation des Virus bzw. die Prozessierung eines synthetisierten langen Virusproteins in kleinere Proteinabschnitte. Beispiele hierfür sind Aciclovir, Valaciclovir, Foscarnet und Peniclovir.
- Immunosuppressive Wirkstoffe
   Durch Zugabe von immunosuppressiven Wirkstoffen, beispielsweise Kortikoiden und/oder anderen oder Immunmodulatoren kann die Reaktion des Hautgewebes auf die Vermehrung der Viren, insbesondere bei bereits vorhandenen Entzündungen, verbessert und optimiert werden.
- Proteine
   Als erfindungsgemäß einsetzbare Proteine sind Proteine zu verstehen, die auf geeignete Weise in den Replikationsmechanismus der Wirtszelle eingreifen. Auf geeignete Weise bedeutet in diesem Zusammenhang, dass die Proteine die Adsorption des Virus an die Wirtszelle, die Injektion der Virus-Nukleinsäure in die Wirtszelle, die Replikation der DNA der Wirtszelle bzw. der Nukleinsäure des Virus, das Prozessieren der Virus-Nukleinsäure oder den Zusammenbau der Viruspartikel zu einem vollständigen Virus hemmen, bevorzugt inhibieren, oder anderweitig in den Vermehrungszyklus des Virus angreifen. Beispiele hierfür sind Protease-Hemmer, Uncoating-Hemmer, Penetrations-Hemmer, reverse Transkriptions-Hemmer und DNA-Polymerase-Hemmer.
- Peptide
   Als erfindungsgemäß einsetzbare Peptide sind beispielsweise Peptide zu verstehen, die auf geeignete Weise die Membranpermeabilität der Wirtszellenmembran beeinflussen, insbesondere erhöhen. Hierdurch kann ein verbesserter Transport von D20 und ggf. der weiteren pharmazeutischen oder der nicht-pharmazeutischen Wirkstoffe der Erfindung in die Wirtszelle erreicht werden. Ein Beispiel hierfür ist Mellitin.
- Nukleinsäuren
   Durch Zugabe von Nukleinsäuren kann parallel zur antiviralen bzw. virustatischen Wirkung des D20 eine Veränderung der Erbinformation der Zellen im Bereich des Wirkortes oder ein gezieltes Ausschalten ("Gene Silencing") bestimmter Gene, beispielsweise der DNA-Polymerase, von Zellen im Bereich des Wirkortes innerhalb des Hautgewebes und dadurch eine Modifikation des Proteoms erreicht werden. Das "Gene Silencing" kann beispielsweise dazu führen, dass die in die DNA-Schadenabwehr involvierten Gene (zum Beispiel p53, BRCA1, BRCA2, ATM, CHK2) abgeschaltet werden und damit die durch D20 an der Vermehrung gehinderten Viren in den Zellen auch langfristig (nach Ende der topischen D20-Verabreichung) nicht mehr in ein latentes Stadium zurückkehren, sondern langfristig an der Expression viraler DNA gehindert werden. Methoden zum Durchführen eines "Gen Silencing" sind dem Fachmann gut bekannt und beispielsweise in Mello C C , Conte D "Revealing the world of RNA interference",in Nature 431, 338-342 (16. September 2004) beschrieben. Bevorzugt handelt es sich bei den Nukleinsäuren um DNA, vorzugsweise Oligonukleotide, Sense- oder Antisense-DNA, natürliche oder synthetische, cDNA, genomische DNA, nackte DNA, einzel- oder doppelsträngige DNA, oder zirkuläre DNA, oder um RNA, vorzugsweise Antisense-RNA, RNAi, siRNA, oder andere zur Interferenz geeignete RNA-Moleküle, die in ihrer Länge nicht beschränkt sind.

Die Konzentration von erfindungsgemäß neben D20 als pharmazeutischem Wirkstoff verwendeten weiteren pharmazeutischen Wirkstoffen bezogen auf die Gesamtlösung einer erfindungsgemäßen Kombination, liegt im Bereich von mindestens 10⁻⁸ M bis mindestens 5 • 10⁻² M, bevorzugt von mindestens 10⁻⁷ M bis 10⁻³ M, am stärksten bevorzugt von mindestens 10⁻⁶ M bis mindestens 10⁻² M. Ein insbesondere bevorzugter Konzentrationsbereich liegt im Bereich von mindestens 10⁻⁹ M bis mindestens 10⁻² M.

Eine ebenfalls bevorzugte Ausführungsform der vorliegenden Erfindung betrifft die Verwendung von D20 zusammen mit mindestens einem weiteren nicht-pharmazeutischen Wirkstoff, wobei dieser ausgewählt ist aus der Gruppe, bestehend aus pharmazeutisch verträglichen anorganischen oder organischen Säuren oder Basen, Polymeren, Copolymeren, Block-Copolymeren, Einfachzucker, Mehrfachzucker, ionische und nicht-ionische Tenside oder Lipiden sowie Mischungen hiervon, Albumin, Transferrin und DNA-Reparatur-Proteinen, wie Kinase-Inhibitoren.

Der Begriff "nicht-pharmazeutischer Wirkstoff' bezeichnet im Sinne der Erfindung jede(s) pharmakologisch verträgliche und therapeutisch sinnvolle Molekül, Substanz oder Verbindung, das/die kein pharmazeutischer Wirkstoff ist, jedoch vorzugsweise zusammen mit mindestens einem erfindungsgemäßen pharmazeutischen Wirkstoff an einen zu behandelnden Organismus verabreicht wird, beispielsweise in einer erfindungsgemäßen Formulierung formuliert, um qualitative Eigenschaften des/der pharmazeutischen Wirkstoffs/Wirkstoffe zu beeinflussen, insbesondere zu verbessern. Bevorzugt entfalten die nicht-pharmazeutischen Wirkstoffe keine oder im Hinblick auf die beabsichtigte Prophylaxe oder Therapie von Virus-basierten Erkrankungen der Haut keine nennenswerte oder zumindest keine unerwünschte pharmakologische Wirkung. Geeignete nicht-pharmazeutische Wirkstoffe sind beispielsweise pharmakologisch unbedenkliche Salze, beispielsweise Natriumchlorid, Geschmackstoffe, Vitamine, z.B. Vitamin A oder Vitamin E, Tocopherole oder ähnliche im menschlichen Organismus vorkommende Vitamine oder Provitamine, Antioxidantien, wie beispielsweise Ascorbinsäure, sowie Stabilisatoren und/oder Konservierungsstoffe zum Verlängern der Verwendungs- und Aufbewahrungsdauer eines pharmazeutischen Wirkstoffes oder einer erfindungsgemäßen Formulierung und sonstige dem Fachmann bekannte, übliche nicht-pharmazeutische Wirkstoffe bzw. Hilfs- und Zusatzstoffe. Weitere erfindungsgemäß bevorzugte nicht-pharmazeutische Wirkstoffe sind insbesondere alle zur Bildung wässriger Gele befähigten Stoffe, wie z.B. natürliche oder synthetische wasserlösliche Polymere, die Netzwerke ausbilden können.

Nachfolgend werden erfindungsgemäß bevorzugte weitere nicht-pharmazeutische Wirkstoffe einer erfindungsgemäßen Kombination und deren Wirkung sowie geeignete Konzentrationen aufgeführt, wobei diese Aufzählung nur beispielhaft ist und die vorliegende Erfindung nicht hierauf beschränkt ist:
- Wasserlösliche Hilfs- und Zusatzstoffe
   Durch Zugabe von wasserlöslichen Hilfs- und Zusatzstoffen, wie z.B. pharmazeutisch verträglichen anorganischen oder organischen Säuren, Basen, Salzen und/oder Puffersubstanzen zur Einstellung des pH Wertes, kann die physiologische Verträglichkeit des D20 auf und in der Haut für nicht Virus-infizierte Zellen verbessert werden.
   Beispiele für bevorzugte anorganische Säuren sind ausgewählt aus der Gruppe, bestehend aus Salzsäure, Bromwasserstoffsäure, Salpetersäure, Schwefelsäure und Phosphorsäure, wobei insbesondere Salzsäure und Schwefelsäure bevorzugt sind. Beispiele für besonders geeignete organische Säuren sind ausgewählt aus der Gruppe bestehend aus Äpfelsäure, Weinsäure, Maleinsäure, Bernsteinsäure, Essigsäure, Ameisensäure und Propionsäure und insbesondere bevorzugt Ascorbinsäure, Fumarsäure und Zitronensäure. Gegebenenfalls können auch Mischungen der genannten Säuren eingesetzt werden, insbesondere von Säuren, die neben ihren Säuerungseigenschaften auch andere Eigenschaften, z.B. in Verwendung als Antioxidantien, besitzen, wie beispielsweise Zitronensäure oder Ascorbinsäure. Beispiele für pharmazeutisch verträgliche Basen sind Alkalihydroxide, Alkalicarbonate und Alkali-Ionen, bevorzugt Natrium. Mischungen dieser Substanzen können insbesondere zur Einstellung und Pufferung des pH Wertes benutzt werden, besonders bevorzugt sind hierbei Kaliumhydrogenphosphat und Di-Kaliumhydrogenphosphat sowie Natriumhydrogenphosphat und Di-Natriumhydrogenphosphat. Bevorzugte Puffersubstanzen im Sinne der Erfindung sind weiterhin PBS, HEPES, TRIS, MOPS sowie weitere physiologisch verträgliche Substanzen mit einem pK Wert im Bereich 5,0 bis 9,0. Die Konzentration dieser Substanzen, bezogen auf die Gesamtlösung einer erfindungsgemäßen Kombination, liegt vorzugsweise im Bereich von Mikromolar bis Millimolar, besonders bevorzugt im Bereich 1- 100 mM.
- Wasserlösliche, polymere Moleküle
   Durch Zugabe von wasserlöslichen, nicht-cytotoxischen Molekülen, wie z.B. bestimmten Polymeren (z.B., aber nicht hierauf beschränkt, Dextran, Polyethylenglykol, Agarose, Zellulose, Acrylsäre, Hylaronsäure), Copolymeren und Block-Copolymeren kann durch deren hohe Wasserbindungskapazität eine zusätzliche Verzögerung (Retardierung) des Übergangs des D20 aus topischen Applikationsformen in die Haut, wie auch von der Haut ins System, erreicht werden. Durch die Fähigkeit der Polymere, das chemische Potential (Wasserpotential) von D20 zu erniedrigen, kann darüber hinaus die Stärke und Richtung des osmotischen Gradienten über die Haut verändert bzw. verbessert und optimiert werden. Die Konzentration dieser Substanzen, bezogen auf die Gesamtlösung, liegt im Bereich von Mikromolar bis Molar, bevorzugt im Bereich 1- 500 mM.
- Wasserlösliche, nicht-polymere Moleküle
   Durch Zusatz von wasserlöslichen, nicht-polymeren Molekülen, welche die Dichte und/oder Viskosität von D20 verändern, beispielsweise, aber nicht hierauf beschränkt, Einfachzucker und Mehrfachzucker, insbesondere Glucose, Sacharose, Dextrose, Maltose, Stärke und Cellulose, können die osmotischen Verhältnisse im Bereich der topischen D20 Applikation sowie der D2O-Transport und die D20- Retention in der Haut verändert bzw. optimiert werden. Die Konzentration dieser Substanzen, bezogen auf die Gesamtlösung einer erfindungsgemäßen Kombination, liegt vorzugsweise im Bereich von Millimolar bis Molar, besonders bevorzugt im Bereich 1,0 mM bis 1,5 M.
- D2O-Grenzflächenspannungs-verändernde Moleküle
   Durch Zugabe von Substanzen, welche die Grenzflächenspannung von D20 verändern, beispielsweise, aber nicht hierauf beschränkt, ionische und nicht-ionische Tensiden oder Lipiden, insbesondere einer Mischung aus Tensiden und Lipiden, kann der Transport des D20 von der topischen Applikation in die Haut sowie innerhalb der Haut verändert werden. Die Konzentration dieser Substanzen, bezogen auf die Gesamtlösung einer erfindungsgemäßen Kombination, liegt vorzugsweise im Bereich von Mikomolar bis Millimolar, besonders bevorzugt im Bereich 0,5 - 500 mM.
- Wasserlösliche nicht-cytotoxische Moleküle
   Durch Zugabe von wasserlöslichen Molekülen, welche dafür bekannt sind, dass sie durch metabolisch besonders aktive Zellen, wie z. B. Virus-infizierten Zellen, in besonderem Maße aufgenommen werden, beispielsweise Albumin oder Transferrin, kann eine zusätzliche Erhöhung der D2O-Transportrate der von einer D20-Hydrathülle umgebenen Molekülen in die Targetzellen der Haut erreicht werden.

Die Konzentration bzw. Dosierung von D20 und ggf. des mindestens einen weiteren pharmazeutischen und/oder nicht-pharmazeutischen Wirkstoffs unterliegt verschiedenen Faktoren, beispielsweise der Art der Behandlung, Art der Erkrankung, Erkrankungszustand des Patienten (Säugetier), Art des Wirkstoffs usw.. Dem Fachmann sind solche Parameter bekannt und die Bestimmung der speziellen Dosierungen unterliegt dem Fachwissen des Fachmanns. Geeignete Konzentrationsangaben werden hierin offenbart. Einige beispielhafte Angaben zu geeigneten Konzentrationsbereichen sind bereits oben aufgeführt, wobei diese lediglich Richtwerte darstellen sollen.

Das erfindungsgemäß verwendbare D20 liegt bevorzugt als Flüssigkeit vor. Vorzugsweise liegt das D20 in einer Lösung, bevorzugt mit H20 (Wasser) als Lösungsmittel, vor und wird hierin auch als "Mischung aus D20 und H2O" oder "D2O/H2O", wenn H2O enthalten ist, bezeichnet, bzw. als "D2O-Lösung" oder "reines D20", wenn kein H2O enthalten ist, bezeichnet. Reines D20 enthält D20 vorzugsweise in einem Konzentrationsbereich von 98,1 bis 100%, bevorzugt von 98,5 bis 99,9%, besonders bevorzugt von 99,7% bezogen auf den Gesamtwassergehalt der Lösung. Eine erfindungsgemäße Mischung aus D20 und H2O enthält D20 vorzugsweise in einem Konzentrationsbereich von 1 bis 98%, bevorzugt von 5 bis 95%, weiterhin bevorzugt von 10 bis 90%, ebenfalls bevorzugt von 15 bis 80%, stärker bevorzugt von 20 bis 70%, ebenfalls stärker bevorzugt von 30 bis 60%, am stärksten bevorzugt von 40 bis 50%, wobei sich diese Angaben auf den Gesamtwassergehalt der Mischung aus D20 und H2O beziehen. Die Herstellung einer erfindungsgemäßen D20-Lösung und analog hierzu einer erfindungsgemäßen Kombination erfolgt beispielsweise durch Mischen der Komponenten, insbesondere von D20 und ggf. H2O sowie ggf. des mindestens einen weiteren pharmazeutischen und/oder nicht-pharmazeutischen Wirkstoffs. Weiterhin kann ggf. ein Lösungsmittel, wie nachfolgend beschrieben, durch Mischen hinzugefügt werden. Vorzugsweise erfolgt die Beimischung von H2O bzw. des mindestens einen weiteren pharmazeutischen und/oder nicht-pharmazeutischen Wirkstoffs bzw. des Lösungsmittels zu D20 im flüssigen Aggregatzustand. Die Herstellung kann jedoch durch jedes beliebige geeignete Verfahren erreicht werden. Im Fall, dass D20 allein (d.h. nicht in Kombination mit weiteren pharmazeutischen und/oder nicht-pharmazeutischen Wirkstoffen) und bei einer Konzentration von 100% bzw. ca. 99.9% bezogen auf das Gesamtvolumen der Lösung (d.h. reines D20) verwendet wird, stellt reines D20 die erfindungsgemäße D20-Lösung dar.

Sämtliche in dieser Beschreibung offenbarten erfindungsgemäßen Verwendungen und (topischen) Applikationen von D20 sind ebenfalls auf eine erfindungsgemäße Mischung aus D20 und H2O uneingeschränkt anwendbar, sofern nichts Gegenteiliges angezeigt ist. Ebenfalls finden Verwendungen einer erfindungsgemäßen Mischung aus D20 und H2O in Bezug auf die erfindungsgemäßen Kombinationen, Schichtsysteme, Pflaster und Bandage, Formulierungen und Aerosole (siehe unten) uneingeschränkt Anwendung sofern nichts Gegenteiliges angezeigt ist.

Die erfindungsgemäße Verwendung von D20 kann, wie nachfolgend näher beschrieben, ebenfalls als Aerosol, Dampf oder Formulierung, insbesondere als Creme, Salbe oder Gel, insbesondere Hydrogel erfolgen. Auf die in diesem Zusammenhang zu verwendenden Konzentrationen von D20 wird ebenfalls nachfolgend eingegangen.

In einer bevorzugten Ausführungsform ist der mindestens eine weitere pharmazeutische Wirkstoff bzw. weitere nicht-pharmazeutische Wirkstoff an D20 gebunden. "Gebunden" im Sinn der vorliegenden Erfindung bedeutet, dass der pharmazeutische bzw. nicht-pharmazeutische Wirkstoff von dem D20 hydratisiert wird.

In weiteren bevorzugten Ausführungsformen ist das D20 bzw. die erfindungsgemäße Kombination in einem geeigneten Lösungsmittel enthalten. Ein erfindungsgemäßes Lösungsmittel kann ein anorganisches oder organisches Lösungsmittel sein. Geeignete Lösungsmittel der vorliegenden Erfindung sollten vorzugsweise von dem Organismus (insbesondere Säugetier), dem der Wirkstoff mit Lösungsmittel verabreicht wird, physiologisch gut verträglich sein, d.h. keine Nebenwirkungen, z.B. toxische Nebenwirkungen, auslösen. Ein insbesondere bevorzugtes Lösungsmittel ist destilliertes Wasser. Ebenfalls bevorzugt sind Ethanol-Wasser-Mischungen; hierbei liegt der prozentuale Massenanteil von Ethanol in diesen Mischungen bevorzugt im Bereich zwischen 5 % und 99 % Ethanol, ebenfalls bevorzugt im Bereich von 10 % bis 96 % Ethanol, stärker bevorzugt zwischen 50 % und 92 %, am stärksten bevorzugt zwischen 69 % und 91 % Ethanol.

D20 oder eine erfindungsgemäße Kombination können "vorformuliert" vorliegen, beispielsweise verpackt in geeigneten Mitteln zum Transport von pharmazeutischen Wirkstoffen, sog. "drug delivery"-Systemen, beispielsweise in Nanopartikeln, Vektoren, bevorzugt Gentransfer-Vektoren, viralen oder nicht viralen Vektoren, Poly- oder Lipoplex-Vektoren, Liposomen oder Hohlkolloide (d.h. Hohlkugeln kolloider Dimension). Weiterhin zum Transport geeignet sind nackte Nukleinsäuren, insbesondere nackte DNA. Geeigneten Vektoren, Liposomen, Hohlkolloide oder Nanopartikel sowie Verfahren zum Einbringen von Substanzen, in solche Vektoren, Liposomen, Hohlkolloide oder Nanopartikel sind allgemein im Stand der Technik gut bekannt und beispielsweise in Cryan S-A., Carrier-based strategies for Targeting Protein and Peptide Drugs to the Lungs, AAPS Journal, 2005, 07(01):E20-E41 und Sambrook et al. Molecular Cloning A Laboratory Manual, Cold Spring Harbor Laboratory (1989) NY beschrieben. Als Gentransfer-Vektoren können vorzugsweise polyethylenimine oder kationische Lipide, wie z.B DOTAP, verwendet werden. Liposomen sind bevorzugt für die Verpackung von Cytostatika verwendbar; eine detaillierte Beschreibung erfolgt beispielsweise in Koshkina NV et al., Koshkina, N.V., et al., Paclitacel liposome aerosol treatment induces inhibition of pulmonary metastases in murine renal carcinoma model., Clinical Cancer Research, 2001, 7, 3258-3262. Proteine als pharmazeutische Wirkstoffe können vorzugsweise mittels superkritischer Flüssigkeiten, Emulsionsverfahren und Sprühtrocknung in biokompatible Poly-Milch/Glykolsäure-Polymere (PLGA) verpackt werden.

Die topische Verabreichung von D20 kann ebenfalls über ein Pflaster oder eine Bandage erfolgen. Eine weitere bevorzugte Ausführungsform betrifft demnach die erfindungsgemäße Verwendung von D20, wobei D20 mit einem bzw. über ein Pflaster oder eine Bandage topisch appliziert wird.

Als "Pflaster" oder "Bandagen" im Sinne der Erfindung, sind alle auf der Haut durch mechanische oder chemische Wechselwirkung, Physisorption, Adhäsion oder andere physikalisch-chemische Prozesse fixierbare Vorrichtungen zu verstehen, welche dazu geeignet sind, einen ausgewählten Hautbereich okklusiv oder nicht-okklusiv über einen für die beabsichtigte Behandlung angemessen langen Zeitraum abzudecken und die Zuführung von D20 an die Haut zu ermöglichen und/oder unterstützen. Erfindungsgemäß anwendbare Pflaster und Bandagen als Applikationssysteme zur lokalen Freisetzung von Wirkstoffen auf der Haut (z.B. Wärmepflaster) sowie zur kontrollierten systemischen Freisetzung von Wirkstoffen (z.B. Opiat-Depotpflaster, Nitroglycerin-Depotpflaster) sind im Stand der Technik bekannt. Als "Depot-Pflaster" oder "Depot-Bandage" soll zusätzlich zu den oben beschriebenen Eigenschaften die Fähigkeit des Pflasters oder der Bandage zur Speicherung von D20 und dessen kontrollierte Abgabe an die Haut über einen Zeitraum von Tagen oder Wochen verstanden werden. Solche Depot-Pflaster bzw. Depot-Bandagen sind nachfolgend von den Begriffen Pflaster bzw. Bandage umfasst.

Insgesamt ist für jede kontrollierte Freisetzung von D20 an die Haut folgendes Problem zu beachten: die Freisetzung aus einer direkt auf die Haut aufgetragenen Flüssigkeit oder Formulierung als Salben, Creme oder Gel kann durch die Richtung des osmotischen Gradienten innerhalb der Haut von innen nach außen erschwert werden, da das D20 in der Flüssigkeit, Salbe, Creme oder in dem Gel unter Umständen ein niedrigeres Wasserpotential aufweist als das H20 in der Haut und in den darunter liegenden Gefäßen.

Eine besonders bevorzugte Ausführungsform der topischen Applikation von D20 ist es daher, durch gezielte Manipulation der osmotischen Verhältnisse im zu behandelnden Hautgebiet die Tiefe bzw. den Grad des Eindringens von D20 in die Haut zu regulieren und damit zu kontrollieren, und zwar vorzugsweise bis zu der Tiefe, in der die hyperproliferierenden Zellen liegen, bevorzugt bis zur Epidermis oder bis zur Dermis. Dies kann durch die gewählte Zusammensetzung einer applizierten erfindungsgemäßen Kombination erreicht werden, indem Substanzen zugefügt werden, welche in der Lage sind, die osmotischen Verhältnisse an der Oberfläche der Haut zu verändern. Beispiele für solche Substanzen sind weiter oben aufgeführt.

Eine weitere Möglichkeit zum kontrollierten Eindringen von D20 in die Haut besteht in der Verwendung von einer oder mehreren Membran(en) oder Folie(n), welche die Passage von Wasser und Gasen ermöglicht / ermöglichen, von größeren Molekülen oder Partikeln (einschl. Bakterien, Viren, Einzeller) jedoch verhindert / verhindern. Beispiele für solche erfindungsgemäß verwendbaren Membranen und Folien sind im Stand der Technik bekannt und haben zahlreiche Anwendungen, wie z.B. in Textilien unter dem Markennamen GORE Tex® oder in der Medizin sogenannte Biofilme oder atmungsaktive Pflaster wie z B. Tegaderm®.

Eine ganz besonders bevorzugte Ausführungsform der Erfindung besteht deshalb in der erfindungsgemäßen Verwendung von D20, wobei das D20 topisch mit einem Pflaster oder einer Bandage appliziert wird, wobei das Pflaster oder die Bandage in Kombination mit mindestens einer Membran oder mindestens einer Folie verwendet wird. Vorzugsweise ist die mindestens eine Membran bzw. die mindestens eine Folie eine mikro- oder nanoporöse Membran bzw. Folie.

In einer besonders bevorzugten Ausführungsform erfolgt die erfindungsgemäße topische Verwendung von D20 über eine spezielle Anordnung. Diese Anordnung besteht aus folgenden Komponenten:
- einer vorstehend beschriebenen mikro- oder nanoporösen Membran oder Folie, welche direkt auf die Haut aufgebracht wird,
- gefolgt von einer D20-Schicht, welche das D20 enthält,
- ggf. gefolgt von einer sog. Okklusionsschicht welche eine Verdampfung des D20 nach außen verhindert oder reguliert und gleichzeitig einen mechanischen Schutz darstellt, welcher das Entweichen von D20 als Flüssigkeit verhindert und
- gefolgt von einem Pflaster oder einer Bandage.

In einer weiteren bevorzugten Ausführungsform erfolgt die erfindungsgemäße topische Verwendung von D20 über eine weitere spezielle Anordnung Diese Anordnung besteht aus folgenden Komponenten:
- einer vorstehend beschriebenen mikro- oder nanoporösen Membran oder Folie, welche direkt auf die Haut aufgebracht wird,
- gefolgt von einer D20-Schicht, welche das D20 enthält,
- ggf. gefolgt von einer sog. Okklusionsschicht welche eine Verdampfung des D20 nach außen verhindert oder reguliert und gleichzeitig einen mechanischen Schutz darstellt, welcher das Entweichen von D20 als Flüssigkeit verhindert und
- gefolgt von einer weiteren vorstehend beschriebenen mikro- oder nanoporösen Membran oder Folie.

Bei Bedarf können selbstverständlich weitere D20-Schichten hinzugefügt werden, die weiteres D20 enthalten, und die von Membranen bzw. Folien separiert werden, wodurch die Depotwirkung oder der Übergang des D20 in die Haut verändert werden kann, beispielsweise die Freisetzungsmenge und/oder -dauer von D20. Der verwendete Begriff "D2O-Schicht" bezieht sich auf flüssiges reines D20, auf eine Mischung aus D20 und H2O sowie auf eine Formulierung von D20, insbesondere als Creme, Salbe, Gel oder Hydrogel.

Ebenfalls können vorzugsweise auch Schichten hinzugefügt werden, die chemische, elektrische oder thermische Eigenschaften aufweisen, welche geeignet sind, den Übergang des D20 in die Haut und/oder die Dauer seiner Freisetzung zu manipulieren. Beispiele hierfür sind Schichten, die geeignet sind, ein elektrisches, thermo-elektrisches, thermisches oder chemisches Potential (oder eine Kombination davon) über die darunter liegenden Schichten und die Haut aufzubauen und/oder aufrechtzuerhalten. Dies kann beispielsweise durch in die beschriebenen Membranen oder Folien eingebettete oder auf ihnen befindliche Elektroden erreicht werden, welche von außen mit Strom (Gleichspannung, Wechselspannung oder hochfrequente Ströme) versorgt werden oder die durch die gezielte Wahl des Elektrodenmaterials mit der D20 Schicht als Elektrolyt elektrochemische Potentiale erzeugen.

Die Gesamtheit solcher vorbeschriebenen D20-Schichten, Okkulsionsschichten, Schichten mit chemischen, elektrischen oder thermischen Eigenschaften, Membranen und Folien in jeder beliebigen, für den Verwendungszweck geeigneten Anzahl, Kombination und Anordnung, wird nachfolgend als "Schichtsystem" bezeichnet. Ein solches Schichtsystem wird vorzugsweise in Verbindung mit einem/einer vorstehend beschriebenen (Depot-) Pflaster oder (Depot-) Bandage verwendet werden.

Durch Variation von Morphologie (Porengröße, Membran- bzw. Foliendicke, Oberflächenrauheit und Oberflächenprofil) und Oberflächeneigenschaften (z.B. hydrophil oder hydrophob, chemischen Beschichtungen -kovalent gebunden oder adhäsiv gebunden-, funktionellen Gruppen, Bindung oder Einlagerung anorganischer oder organischer Substanzen) der direkt im Kontakt mit der Haut befindlichen Membran oder Folie kann der Übergang des D20 aus einem beschriebenen Pflaster, Bandage oder Schichtsystem in die Haut gezielt beeinflusst bzw. verändert werden.

Eine weitere Variation des Eintritts von D20 in die Haut ist durch die gezielte Verwendung von Klebstoffen möglich, welche zur mechanischen Befestigung des (Depot-) Pflasters oder der (Depot-) Bandage auf der Haut verwendet werden können, aber nicht zwingend notwendig sind. Die allgemein für topische Anwendungen von Pflastern und Bandagen verwendeten Klebstoffe weisen eher hydrophoben Charakter auf, welcher den Durchtritt von D20 durch die Klebstoffschicht verhindert kann. Durch Zumischen von Zusatzstoffen in die Klebstoffzubereitung kann eine Veränderung dieser Eigenschaften erreicht werden. Als solche "Zusatzstoffe" kommen organische und/oder anorganische Substanzen und Verbindungen in Betracht, welche in der Lage sind, die Permeationseigenschaften von D20 durch die Klebstoffschicht zu verändern. Beispiele für solche Substanzen sind u.a. Polymere, Co-Polymere, Blockpolymere, Block-Co-Polymere, Tenside, Peptide, Proteine, Nukleinsäuren, Sterole und Steroide.

In einer weiteren bevorzugten Ausführungsform erfolgt die erfindungsgemäße Verwendung von D20, vorzugsweise mit einem Pflaster oder einer Bandage, topisch auf die Haut über eine Anordnung, die den Übergang von D20 in die Haut weitgehend oder ausschließlich über die Dampfphase ermöglicht. D.h. erfindungsgemäß als Flüssigkeit verwendetes D20 verdampft als molekulares D20 und kommt als Dampf mit der Haut in Kontakt. Die Konzentrationen des D20 entsprechen damit den oben beschriebenen Konzentrationen einer D20-enthaltenden Flüssigkeit. Dampfförmiges D20 hat den Vorteil eines besonders leichten Eindringens in die Haut. Um dieses Verdampfen zu bewirken, ist thermische Energie erforderlich, die entweder von der Haut selbst oder von einer externen Wärmequelle, z.B. bei Verwendung eines beschriebenen Pflasters oder Bandage oder oben beschriebenen Schichtsystems hierin eingebrachten elektrischen Heizung (z.B. Peletier-Heizung), bezogen werden kann. Zusätzlich zu dieser thermischen Energie kann durch gezielte Modifikationen, beispielsweise der Wahl der Morphologie (insbesondere Porengröße und Oberflächenbeschichtung), einer ersten, auf der Haut befindlichen oben beschriebenen Membran oder Folie des Schichtsystems erreicht werden, dass nur dampfförmiges D20 durch die Membran oder Folie bis zur Haut vordringen kann, flüssiges D20 hingegen zurückgehalten wird. Auch bei dieser Ausführungsform lassen sich durch die oben beschriebenen Veränderungen des Schichtsystems und entsprechenden Modifikationen an ggf. weiteren verwendeten Membranen oder Folien, die Menge und die Dauer der Freisetzung von D20 über die Dampfphase kontrolliert und/oder verändert werden.

Eine weitere bevorzugte Ausführungsform der Erfindung betrifft die Verwendung von D20, wobei das D20 als Aerosol topisch appliziert wird.

Unter einem "Aerosol" versteht man feste oder flüssige schwebende Partikel mit einem Durchmesser von ca. 0,0001 µm bis ca. 100 µm, in Gasen insbesondere Luft, wobei Zusammensetzung und Form der Aerosole sehr stark variieren kann. Die kleinsten pharmazeutisch wirksamen Partikel in Aerosolen sind beispielsweise Nukleinsäuren, Peptide oder Proteine, die größten Partikel beispielsweise Nebelpartikel. Häufig bestehen Aerosole aus Gemischen von Partikeln unterschiedlicher Partikelgrößen und verkörpern dadurch eine polydisperse Größenverteilung. Aerosole lassen sich künslich durch im Stand der Technik gut bekannte Dispersions- und Kondensationsverfahren herstellen. Sie können ohne Treibgas verwendet werden oder in Verbindung mit einem verflüssigten Druckgas als Treibgas, beispielsweise in Sprühdosen, verwendet werden.

Die erfindungsgemäße Verwendung von D20 als Aerosol erfolgt bevorzugt über einen Vernebler. Als "Vernebler" für die vorliegende Erfindung ist jeder beliebige für medizinische Aerosole geeignete Apparat zu verstehen, mit dem Aerosolpartikel im Größenbereich 50 nm bis 50 µm produziert werden können. Der Vernebler versprüht ein definiertes Volumen des D20, meist unter Anwendung hoher Drücke durch kleine Düsen, um so ein auf der Haut applizierbares, Aerosol zu erzeugen.

Geeignete Vernebler für umfassen auch Treibgas-getriebene Inhalationsgeräte bzw. Vernebler. Treibgase können hierbei beispielsweise FCKW oder HFKW sein. Diesbezüglich wird auf "Theorie und Praxis der Inhalationstherapie", Seiten 31 bis 70Arcis Verlag (2000) verwiesen, wo eine ausführliche Beschreibung verwendbarer Vernebler sowie Verfahren zu deren Anwendung offenbart ist/sind.

Beispiele für erfindungsgemäß geeignete Vernebler sind pressluftgetriebene Düsenvernebler (z.B. PARI LC plus, PARI GmbH, Starnberg, Deutschland), Venturi-Düsenvernebler, Wasserdampfgetriebene Düsenvernebler oder Ultraschallvernebler (z.B. AeronebLab, Aerogen, Inc., Stierlin Court, Canada; eFLOW, PARI GmbH, Starnberg, Deutschland). Ebenfalls geeignet sind Vernebler mit einer Größe, die von dem Patienten (Mensch) mitgeführt werden können, z.B. der Respimat@, wie in WO 97/12687, beschrieben. Sämtliche hierin zitierten Referenzen sind vollumfänglich in die vorliegende Erfindung einbezogen.

Die Herstellung eines erfindungsgemäß verwendbaren Aerosols kann anhand geeigneter bekannter Standardtechniken zur Aerosolherstellung erfolgen.

Die für die erfindungsgemäße Verwendung von D20 als Aerosol zu verwendenden D20-Konzentrationen ist von verschiedenen Faktoren abhängig, beispielsweise dem Verwendungszweck, Erkrankungszustand und unterliegen dem Fachwissen des Fachmanns. Ein erfindungsgemäß verwendbares Aerosol enthält D20 vorzugsweise in einem Konzentrationsbereich von 1 bis 98 Gew.-%, bevorzugt von 10 bis 90 Gew.-%, ebenfalls bevorzugt von 15 bis 80 Gew.-%, stärker bevorzugt von 20 bis 70 Gew.-%, ebenfalls stärker bevorzugt von 30 bis 60 Gew.-%, am stärksten bevorzugt von 40 bis 50 Gew.-%.

Die Verabreichung für die erfindungsgemäße Verwendung von D20 als Aerosol erfolgt vorzugsweise unmittelbar auf die Haut im zu behandelnden Gebiet. Dies kann z.B. durch eine auf der Haut aufsetzbare und zu dieser hin geöffneten Kammer erfolgen, durch welche das Aerosol geleitet wird.

Eine weitere bevorzugte Ausführungsform betrifft die erfindungsgemäße Verwendung von D20, wobei D20 als Formulierung topisch appliziert wird. Vorzugsweise ist eine solche Formulierung eine Salbe, Creme oder Gel, insbesondere Hydrogel.

Unter einer "Salbe" gemäß der vorliegenden Erfindung ist eine äußerlich zu verwendende Arzneimittelzubereitung aus einer Grundmasse schmierfähiger Stoffe, wie Vaseline, zu der die eigentlichen pharmazeutischen und/oder nicht pharmazeutische Wirkstoffe zugegeben werden, beispielsweise durch Mischen.

Unter einer "Creme" im Sinne der vorliegenden Erfindung ist eine erfindungsgemäße Salbe zu verstehen, die zusätzlich weitere Bestandteile enthalten kann, wie kosmetische Wirkstoffe, z.B. Duftstoffe, Farbstoffe und/oder Emulgatoren, z.B. Lecithin. Von einer Creme wird im allgemeinen eine Lotion unterschieden, wobei diese Unterscheidung meistens abhängig von dem Grad der Viskosität gemacht wird. Erfindungsgemäß ist unter einer Creme auch eine Lotion zu verstehen.

Ein "Gel" im Sinne der vorliegenden Erfindung ist die Lösung einer makromolekularen Substanz, z. B. Agarose, Acrylsäure, Alginsäure, Polysiloxane oder Acrylamid, deren Konzentration so hoch ist, dass sich die gelösten Makromoleküle unter geeigneten Bedingungen und ggf. unter Zugabe weiterer Substanzen (z. b. Salze, Säuren, Füllstoffe, Puffersubstanzen) zu einem schwammartigen, dreidimensionalen Gerüst verbinden, in dessen Hohlräumen sich eine Flüssigkeit befindet. Dadurch haben Gele eine relativ feste Konsistenz. Die Viskosität liegt zwischen flüssig und fest. Eine solche Flüssigkeit ist bevorzugt reines D20 oder eine Mischung aus D20 und H2O.

Als "Hydrogel" im Sinne der Erfindung wird ein Gel bezeichnet, welches durch besonders hohe Aufnahmekapazität von Wasser gekennzeichnet ist, im Sinne der Erfindung besteht es bevorzugt zu 20-99%, stärker bevorzugt zu 70-99%, und besonders bevorzugt zu 80-99%, aus Wasser, ohne jedoch die rheologischen Eigenschaften einer klassischen Flüssigkeit zu zeigen. In einer besonders bevorzugten Ausführungsform ist das Hydrogel transparentdurchsichtig und gleichzeitig streichfähig, ohne das seine Morphologie und Integrität durch das verstreichen des Gels beeinträchtigt wird.

Die Herstellung einer erfindungsgemäß verwendbaren Formulierung, insbesondere einer Salbe, Creme oder einem Gel, ist exemplarisch in den Beispielen beschrieben. Sofern eine solche Formulierung weitere pharmazeutische und/oder nicht-pharmazeutische Wirkstoffe enthalten, werden diese vorzugsweise durch Mischen der Formulierung hinzugefügt. Sie kann jedoch nach jedem beliebigen im Stand der Technik bekannten Standardverfahren erfolgen. Dem Fachmann sind solche Verfahren und ebenfalls die zu wählenden Konzentrationen der zu verwendenden Komponenten bzw. Substanzen bekannt.

Die Konzentrationen von D20 in einer erfindungsgemäß verwendbaren Formulierung liegen vorzugsweise in folgenden Bereichen:
- für eine Creme oder Salbe bevorzugt im Bereich von 0,1 bis 98 Gew.-%, bevorzugt von 5 bis 85 Gew.-%, ebenfalls bevorzugt von 10 bis 80 Gew.-%, besonders bevorzugt von 15 bis 70 Gew.-%, stärker bevorzugt von 20 bis 60 Gew.-% und am stärksten bevorzugt von 25 bis 50 Gew.-% und
- für ein Gel bevorzugt 0,1 bis 99,8 Gew.-%, bevorzugt von 10 bis 99 Gew.-%, ebenfalls bevorzugt von 15 bis 80 Gew.-%, besonders bevorzugt von 20 bis 70 Gew.-%, stärker bevorzugt von 30 bis 70 Gew.-% und am stärksten bevorzugt von 35 bis 65 Gew.-%.

Der Fachmann wird insbesondere abhängig von der vorliegenden Indikation, dem Zustand des zu behandelnden Organismus (Patienten), der Schwere der Erkrankung usw. die geeignete Konzentration wählen.

In einer besonders bevorzugten Ausführungsform enthält eine erfindungsgemäß verwendbare Formulierung weiterhin mindestens ein anorganisches oder organisches Lösungsmittel. Bevorzugt ist das Lösungsmittel ausgewählt ist aus der Gruppe, bestehend aus Ethanol, Wasser und Glycerol sowie Mischungen davon.

Sämtliche in dieser Beschreibung offenbarten erfindungsgemäßen Verwendungen und Verabreichungen von D20, beispielsweise mit Pflastern und Bandagen, Schichtsystemen, finden ebenfalls auf die erfindungsgemäß verwendbaren Formulierungen uneingeschränkt Anwendung, sofern nichts Gegenteiliges angezeigt ist.

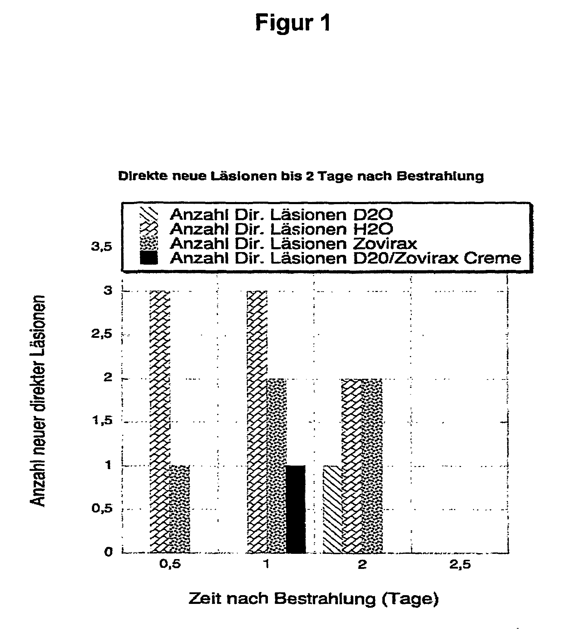

Die vorliegende Erfindung wird nachfolgend anhand von Beispielen weiter erläutert, wobei diese die Gegenstände der Erfindung nicht beschränken.Die vorliegende Erfindung wird ebenfalls anhand der Figuren 1 bis 3 illustriert, wobei diese die Gegenstände der Erfindung nicht beschränken.
**Figur 1** ist eine Darstellung auftretender direkter neuer Läsionen bis 2 Tage nach Bestrahlung. Die Anzahl direkter neuer Läsionen im Zeitraum bis zu 2 Tagen nach UV-Bestrahlung für 4 Versuchsgruppen von je 8 Testpersonen, die beginnend nach der Bestrahlung wie folgt behandelt wurden: alle 2,5 Stunden (außer nachts) wurde der bestrahlte Lippenbereich bei jeweils 8 Testpersonen mit D20-Carbopolsäuregel (hergestellt nach Beispiel 1 mit 100% D2O), mit H2O-Carbopolsäuregel (Placebo, hergestellt nach Beispiel 1 mit 100% H2O), mit Zovirax Creme (Aciclovir im Cremespender von GlaxoSmithKlihe) oder mit einer nach Beispiel 7 hergestellten D20-Creme bestrichen, in welche zusätzlich 20 Gew% Zovirax Creme homogen eingemischt war, bestrichen.

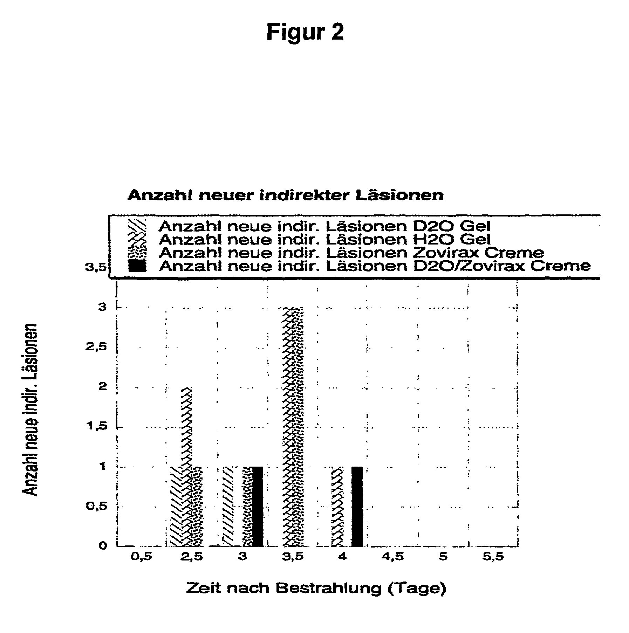

**Figur 2** ist eine Darstellung auftretender neuer indirekter Läsionen. Die Anzahl neuer indirekter Läsionen im Zeitraum bis zu 15 Tagen nach der UV Bestrahlung für 4 Versuchsgruppen von je 8 Testpersonen, die beginnend nach der Bestrahlung wie folgt behandelt wurden (Beispiel 11): alle 2,5 Stunden (außer nachts) wurde der bestrahlte Lippenbereich bei jeweils 8 Testpersonen mit D20 Gel (Hergestellt nach Beispiel 1 mit 100% D20), mit H2O Gel (Placebo, Hergestellt nach Beispiel 1 mit 100% H2O), mit Zovirax Creme (Aciclovir im Cremespender von GlaxoSmithKline) oder mit einer nach Beispiel 7 hergestellten D20 Creme bestrichen, in welche zusätzlich 20 Gew% Zovirax Creme homogen eingemischt war, bestrichen.

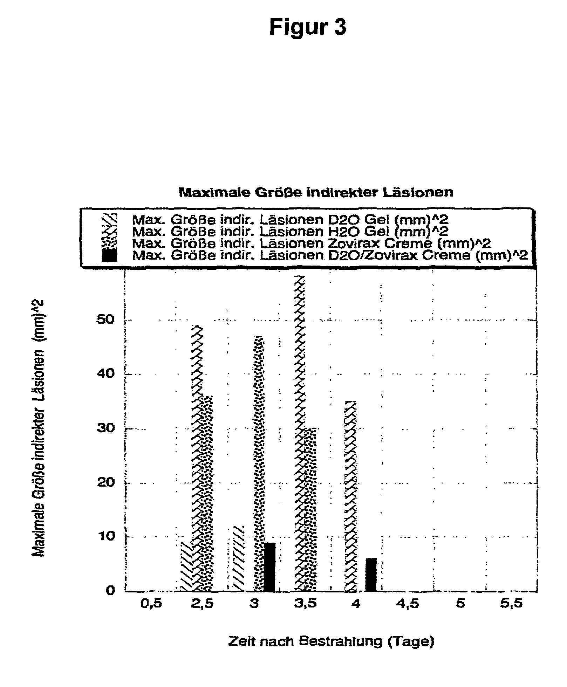

**Figur 3** ist eine Darstellung der maximalen Größe auftretender indirekter Läsionen. Die maximale Größe der in Figur 2 dargestellten indirekten Läsionen für die 4 Versuchsgruppen. Die dargestellten Werte für den Flächenbedarf der Läsionen sind die Mittelwerte aus den für die jeweilige Versuchgruppe bestimmten maximalen Flächen der einzelnen indirekten Läsionen.

### Beispiele

### Beispiel 1: Herstellung von Hydrogelen auf Basis von Acrylsäure

Es wurde 2,0 Gew.-% Carbopol 980 (Hersteller: Noveon, Inc. , 9911 Brecksville Rd., Cleveland, OH 44141-3247, USA) in getrennten Ansätzen in reinem D20, in reinem H2O oder in einer Mischung aus D20 und H2O durch Rühren gelöst und anschließend durch Pipettieren von 10 M NaOH Lösung auf einen pH Wert von 6,8 titriert. Anschließend wurden die durch die NaOH Zugabe infolge Quervernetzung der Polyacrylsäure über ihre Carboxylgruppen mit den alkalischen Hydroxyl-Gruppen entstandenen farblosen, transparenten und optisch klaren Acrylsäuregele (Carbopolgele) (D20-Carbopolgel, H2O-Carbopollgel, D2O/H2O-Carbopollgel) bei Raumtemperatur bis zur weiteren Verwendung, für mindestens 24 Stunden, gelagert. Das in diesem und allen folgenden Beispielen verwendete D20 der Firma Sigma-Aldrich (München) wies eine Isotopenreinheit von 99,0% auf.

### Beispiel 2: Herstellung von Hydrogelen auf der Basis von Siloxanen (Silicon)

Es wurde 3,0 Gew-% Hexamethyldisiloxane (Handelsname SILMOGEN CARRIER von DOW Corning) und 1 Gew-% Ethanol in getrennten Ansätzen in reinem D20, in reinem H2O oder in einer Mischung aus D20 und H2O durch Rühren gelöst. Diese Lösungen wurden anschließend sofort im Gewichtsverhältnis 1:2 (Siliconlösung:Carbopolgel) unter kräftigen Rühren mit dem nach Beispiel 1 hergestellten Gel (Carbopolgel) gemischt, bis optisch transparente Gele (Silicongele) (D20-Silicongel, H2O-Silicongel, D2O/H2O-Silicongel) entstanden waren. Die Lagerung der Gele erfolgte bei Raumtemperatur bis zur weiteren Verwendung, für mindestens 24 Stunden.

### Beispiel 3: Herstellung von Hydrogel auf der Basis von Alginaten

Es wurde 4,0 Gew-% Alginsäurenatriumsalz (Na-Alginat) (Hersteller: Röhm GmbH Darmstadt, Deutschland) in getrennten Ansätzen in reinem D20, in reinem H2O oder in einer Mischung aus D20 und H2O durch Rühren dispergiert und anschließend durch Pipettieren von 10 M NaOH Lösung auf einen pH Wert von 7,0 titriert. Die entstandenen gelblichbraunen, transparenten Gele (Alginatgele) (D2O-Alginatgel, H2O-Alginatgel, D20/H2O-Alginatgel) wurden bei Raumtemperatur bis zur weiteren Verwendung, für mindestens 24 Stunden, gelagert.

### Beispiel 4: Herstellung von Hydrogel auf der Basis von PVA

Es wurde 20 Gew-% Polyvinylalkohol (PVA C-25, Shin-Etsu Chemical Co., Japan) in getrennten Ansätzen in reinem D20, in reinem H2O oder in einer Mischung aus D20 und H2O durch Rühren gelöst. Anschließend wurden die Lösungen 5 Gefrier-Auftau-Zyklen ("freeze-thaw-cycles") unterworfen. Das Ergebnis waren Gele (PVA-Gele) (D20-PVA-Gel, H2O-PVA-Gel, D2O/H2O-PVA-Gel) mit gummiartigen Eigenschaften, welches in 2 mm dünne Scheiben geschnitten wurde. Die Gele wurden bei Raumtemperatur bis zur weiteren Verwendung, für mindestens 24 Stunden, gelagert.

### Beispiel 5: Herstellung von Hydrogel - Folien bzw. Platten auf der Basis von Agarose

Es wurde 3,0 Gew.-% Agarose in getrennten Ansätzen in reinem D20, in reinem H2O oder in einer Mischung aus D20 und H2O gelöst und die Lösungen anschließend auf 90°C erhitzt. Das verwendete D20 der Firma Sigma-Aldrich (München, Deutschland) wies eine Isotopenreinheit von 98,5% auf. Die heißen Lösungen wurde in geeignete Petrischalen auf 1,0 - 1,5 mm Höhe gegossen und abgekühlt. Die so erhaltenen Gele (Agarosegele) (D2O-Agarosegele, H2O-Agarosegele, D2O/H2O-Agarosegele) wurden unter sterilen Bedingungen bei 4 °C gelagert.

### Beispiel 6: Herstellung von Hydrogel - Folien bzw. Platten auf der Basis von Acrylamid

Es wurden Acrylamidgele (5% Acrylamid) hergestellt, wobei in getrennten Ansätzen reines D20, reines H2O oder eine Mischung aus D20 und H2O vor Zugabe des Acrylamids (mit 2,4% bis-Acrylamid) entgast und auf 40 °C erwärmt wurde. Nach Zugabe von Acrylamid und bis-Acrylamid wurden die Lösungen gemischt (Vortex Mischer, 1 min. bei 200 U/min) und anschließend die Katalysatoren Tetramethyl-ethylendiamin (TEMES ; 1,0%) und Ammoniumpersulfat (AP ; 0,1%) zugegeben, gefolgt von 10 Sek. Mischen. Dann wurden die Gele in Petrischalen gegossen (Höhe des Gels 1,0-1,5 mm) und für 2 Stunden bei 40° C gelagert. Anschließend wurden die Gele (D20-Acrylamidgel, H2O-Acrylamidgel, D20/H2O-Acrylamidgel) gewaschen, wobei zum Waschen das analoge Wassergemisch wie zum Hydratisieren des Gels (reines D20, reines H2O oder eine Mischung aus D20 und H2O) verwendet wurde. Die Gele wurden bei Raumtemperatur bis zur weiteren Verwendung, für mindestens 24 Stunden, gelagert.

### Beispiel 7: Herstellung einer D2O-haltigen Creme

Zu 50 Gramm Asche Basiscreme (Hersteller: Asche Chiesi GmbH, Hamburg, Deutschland) wurde bei 40 °C unter ständigen Rühren langsam D20 hinzugegeben, bis ein Gewichtsanteil vom 38% D20 (bezogen auf das Ausgangsgewicht der Creme) in der homogenen Mischung erreicht war. Die Creme wurde anschließend auf Raumtemperatur abgekühlt und luftdicht verschlossen gelagert.

### Beispiel 8: Herstellung eines D2O-haltigen Aerosols

Für die Erzeugung von Aerosolen wurden ausschließlich Geräte verwendet, die Stand der Technik sind. Verwendet wurde ein Pari LC Plus Universal Vernebler (PARI GmbH, 82319 Starnberg, Deutschland) in Kombination mit einem Pari Universal Kompressor der 200 mg/min polydisperses Aerosol mit einer mittleren Partikelgröße (medianer Massendurchmesser) von 2,5 µm für reines H2O und D20 und von 2.5 - 4,5 µm für H2O bzw. D20 erzeugte (Betriebsdruck 2,0 bar, Durchflussmenge der Kompressorluft war 6,0 l/min.) Die Partikelgrößenmessung erfolgte mittels dynamischer Lichtstreuung in einer Durchfluß-Küvette Die Aerosolerzeugung erfolgte bei einer Temperatur von 37 °C durch entsprechende Thermostatierung des Verneblers in einem Wasserbadthermostaten.

### Beispiel 9: Wirksamkeit im Zellkulturversuch

Menschliche Keratinozyten Primärkulturen, hergestellt nach Reinl, H. M. et al., (J.Invest. Dermatol. 1995; 105(2); 291-5) wurden nach Ausbildung eines geschlossenen Monolayers in der Zellkulturschale und der Ausbildung von Desmosomen mit Zellkulturmedium inkubiert, welches zusätzlich 35 Gew.% D20 (Probe) bzw. H2O (Kontrolle) enthielt. Am Tag nach der Inkubation wurden Probe und Kontrolle mit HSV-1 infiziert. Die Infektion erfolgte durch Zugabe des Virus in Zellkulturmedium (10⁷ Viren pro Zellkulturschale in 1 ml Zellkulturmedium). Nach Ablauf von 3 Tagen (nach Infektion) wurden Probe und Kontrolle auf die mittlere Anzahl von Viren pro Zelle untersucht. Die Viruszählung erfolgte mittels Transmissions-Elektronenmikroskopie nach Standard Fixier- und Färbeverfahren (Glutaraldehyd und Osmiumtetroxid). Es wurden jeweils 20 willkürlich ausgewählte Keratinozytenzellen aus Proben- und Kontrollgruppe bezüglich der in ihnen enthaltenen Viruspartikel ausgewertet.

Die daraus erhaltene mittlere Viruszahl war 10±4 für die Probengruppe (D20) und 28±4 für die Kontrollgruppe (H2O).

### Beispiel 10: Wirksamkeit im Tierversuch

Die Rücken von 12 Meerschweinchen wurden mittels eines Vakzinierungsinstruments mit HSV-1 infiziert, wie es im Detail von McKeogh M B et. al. (Arch. Dermatol. 2001, 137, 1153-1158) beschrieben wurde. Unmittelbar nach der Infektion wurden die Meerschweinchen in 4 randomisierte Gruppen zu je 3 Tieren eingeteilt. Probengruppe 1 erhielt alle 3 Stunden ein nach Beispiel 1 mit 100% D20 hergestelltes Carbopolsäuregel auf den Rücken (auf ca. 12 cm² Fläche) aufgebracht (ca. 1,0 Gramm Gel pro Applikation). Probengruppe 2 erhielt ausschließlich Trinkwasser, welchem 35% D20 beigemischt war, und keine weitere Behandlung. Die Kontrollgruppe 1 erhielt eine analoge Menge Carbopolsäuregel zu identischen Zeitpunkten auf den Rücken aufgetragen, welches nach Beispiel 1 aus 100% H2O hergestellt worden war (H2O-Carbopolgel). Die Kontrollgruppe 2 wurde nach der Infektion keinerlei weiterer Behandlung unterzogen. Nach 5 Tagen wurden die Tiere getötet und die Schwere der Infektion durch Bestimmung der Anzahl und Größe von Läsionen (Papeln und Ulcerationen) untersucht. Außerdem wurde die mittlere Anzahl von HSV-1 pro Keratinozytenzelle (Rücken) elektronenmikroskopisch, wie in Beispiel 9 beschrieben, gemessen.

Das Ergebnis ist in **Tabelle 1** dargestellt und zeigt deutlich die überlegene Wirkung von topisch applizierten D20 (Probengruppe 1) im Vergleich zu H2O bzw. keiner Behandlung (Kontrollgruppen 1 und 2) bezüglich der Hemmung der Läsionenzahl und ihrer Fläche sowie der Anzahl von HSV-1 pro Zelle. Eine virustatische Wirkung von D20 im Trinkwasser (Probengruppe 2) ist ebenfalls nachweisbar, aber nicht so ausgeprägt wie die Wirkung der topischen D20 Applikation (Probengruppe 1).

### Beispiel 11: Wirksamkeit zur HSV-1 Therapie im klinischen Versuch, Vergleich mit Aciclovir

Es wurden 32 gesunde Freiwillige im Alter von 23-58 Jahren (16 weiblich, 16 männlich) für diese Studie ausgewählt. Alle ausgewählten Testpersonen hatten eine Historie von Herpes labialis, welcher insbesondere durch Sonnenlicht (UV-Strahlung) ausgelöst wird. Keine der Testpersonen benutzte eine Woche vor oder während des auf 10 Tage begrenzten Tests antivirale Therapeutika oder immunmodulierende Therapeutika.

Für alle Testpersonen wurde zunächst die individuelle minimale erythematöse Dosis (MED) bestimmt, indem 6 jeweils 1 cm² große Flächen auf dem Oberarm der jeweiligen Testperson für 1 - 6 Minuten mit UV-Licht bestrahlt wurden (Zeitinkrement zwischen der Bestrahlung der 6 Flächen jeweils 1 Min., die jeweils nicht bestrahlten Flächen wurden abgedeckt). Als UV-Lichtquelle wurden zwei Fluoreszenzlampen Typ FS 20 von Westinghouse, Bloomfield, NJ, USA verwendet, die abgegebene Strahlungsleistung wurde vor Applikation an jeder Versuchspeson mit einen Strahlungsmesser (Radiant Power Meter Typ 70260 mit Messkopf Typ 70268 von Newport, Irvine, CA, USA) überprüft. Während der Bestrahlung befand sich die UV-Lichtquelle in 20 cm Abstand von der zu bestrahlenden Hautfläche. Die bestrahlten Flächen der Testpersonen wurden nach 24 Stunden begutachtet und die MED wurde als die kürzeste Zeit festgelegt, nach welcher eine bestrahlte Fläche einen distinkten Rand des Erythems nach 24 Stunden aufwies.

Nach Bestimmung der individuellen MED's wurde die Lippenfläche der Versuchspersonen in 4 Bereiche eingeteilt (obere Lippe rechts bzw. links sowie untere Lippe rechts bzw. links). Eine UV-Bestrahlung erfolgte nur in den Bereichen, welche von der Versuchsperson zuvor als jene mit den häufigsten Herpes labialis Rezidiven identifiziert worden war. Der nicht zu bestrahlende Teil der Lippen wurde mit einer Sonnenschutzcreme (Sonnenschutzfaktor 50) vor der Bestrahlung geschützt. Der zu bestrahlende Lippenbereich erhielt eine Bestrahlungsdosis der vierfachen MED, die für die jeweilige Testperson bestimmt wurde. Die Haut um die zu bestrahlenden Lippen wurde durch eine Maske bzw. durch Sonnenschutzcreme (Sonnenschutzfaktor 50) geschützt, dabei wurde ein Hautstreifen von 0,8 - 1,0 cm ab Lippenrand mit bestrahlt. Anschließend wurden die 32 Testpersonen randomisiert in 4 Gruppen zu je 8 Personen aufgeteilt.

Die 4 Versuchsgruppen wurden unterschiedlich behandelt: Unmittelbar nach der Bestrahlung und von diesem Zeitpunkt an alle 2,5 Stunden (außer nachts) wurde der bestrahlte Lippenbereich bei jeweils 8 Testpersonen mit D20-Carbopolgel (hergestellt nach Beispiel 1 mit 100% D2O), mit H2O-Carbopolgel (Placebo, hergestellt nach Beispiel 1 mit 100% H2O), mit Zovirax Creme (Aciclovir im Cremespender von GlaxoSmithKline) oder mit einer nach Beispiel 7 hergestellten D20-Creme bestrichen, in welche zusätzlich 20 Gew% Zovirax Creme homogen eingemischt wurde, bestrichen.

Die Testpersonen wurden an den Tagen 2, 3, 5, 7, 9, und 15 nach der Bestrahlung untersucht. Dabei wurde die Zahl neuer Läsionen (Bläschen und Ulcerationen) sowie deren maximale Ausdehnung bestimmt. Die Flächenberechung der maximalen Ausdehnung erfolgte unter Annahme einer Kreisform, deren mittlerer Durchmesser aus jeweils 3 Messungen der Läsion ermittelt wurde. Versuchspersonen, die neue Läsionen aufwiesen, wurden für die folgenden 4 Tage nach deren Feststellung täglich auf weitere neue Läsionen untersucht und die Größe aller Läsionen gemessen. Die Läsionen wurden in folgende Klassen unterteilt:
- direkte Läsionen, sie entstanden innerhalb von 48 h nach der Bestrahlung,
- indirekte Läsionen, sie entstanden nach Ablauf dieser Zeit von 48 Stunden und
- reduzierte Läsionen, sie wurden als solche definiert, welche zu jedem Zeitpunkt entstanden, sich aber nicht über eine gerade noch wahrnehmbare Erhebung der betroffenen Haut (Papeln) hinaus entwickelten.

**Figur 1** zeigt die Anzahl direkter Läsionen im Zeitraum von bis zu 2 Tagen nach der Bestrahlung für die 4 Versuchsgruppen. **Figur 2** zeigt die Anzahl indirekter Läsionen im Zeitraum von 2 bis 15 Tagen nach der Bestrahlung für die 4 Versuchsgruppen. **Figur 3** zeigt die maximale Größe der indirekten Läsionen für die 4 Versuchsgruppen als Funktion des Tages, an dem das Maximum festgestellt wurde. **Tabelle 2** listet die Gesamtzahl beobachteter direkter bzw. indirekter Läsionen für die 4 Versuchsgruppen auf.

Zusammenfassend kann man aus den Ergebnissen eine deutliche und nachhaltige Wirkung von D20 zur Unterdrückung von Läsionen im Vergleich zum Placebo (H2O) schlussfolgern. Darüber hinaus ist diese Wirkung von D20 einer handelsüblichen Aciclovir Creme (Zovirax), sowohl bei der Anzahl der Läsionen als auch bei deren maximaler Ausdehung, deutlich überlegen. Auch eine D20 Creme mit 20% Zovirax zeigt eine Wirkung, die in beiden Bereichen (Läsionenzahl und Ausdehnung) der reinen, handelsüblichen Zovirax Creme deutlich überlegen ist. Bei keiner der 4 Versuchsgruppen wurden Nebenwirkungen der Therapie mit den verwendeten pharmazeutischen Zubereitungen beobachtet.

### Beispiel 12: Wirksamkeit von D20 zur HSV-2 Therapie im klinischen Versuch

Es wurden 10 gesunde Freiwillige im Alter von 21- 60 Jahren (6 weiblich, 4 männlich) für diese Studie ausgewählt. Alle ausgewählten Testpersonen hatten eine Historie von rezidivierenden Genitalherpes (HSV-2), bestätigt durch PCR-Tests. Keine der Testpersonen benutzte vier Wochen vor oder während des auf 6 Monate begrenzten Tests antivirale Therapeutika oder immunmodulierende Therapeutika. Die mittlere Zahl von HSV-2 Rezidiven pro Jahr betrug nach Angaben der einzelnen Testpersonen 4,2 ± 2,5 (Standardabweichung über alle 10 Testpersonen). Die Testpersonen wurden in 2 Gruppen (eine Probengruppe, eine Kontrollgruppe) zu je 5 Personen randomisiert und single-blind aufgeteilt.

Die Probengruppe erhielt ein nach Beispiel 2 hergestelltes D20-Silicongel mit 100% D20, die Kontrollgruppe erhielt nach Beispiel 2 hergestelltes H2O-Silicongel mit 100% H2O. Beide Gruppen wurden angewiesen, bei kleinsten Anzeichen einer Rezidivbildung, d.h. initiale Ausbildung von Läsionen (Papeln bzw Ulcerationen), das jeweilige Gel alle 3 Stunden (außer nachts) für die Dauer von 5 Tagen auf die betroffenen Stellen aufzutragen und von diesem Zeitpunkt des ersten Auftragens an die Dauer der Läsion (Rezidivdauer) in Tagen zu messen. Es wurden ausschließlich Ereignisse gezählt, bei denen es mindestens zur fühlbaren Ausbildung einer Läsion kam. Nach 6 Monaten wurde der Versuch beendet und die Berichte der Testpersonen ausgewertet.

Ergebnisse:
a) Kontrollgruppe (H2O-Silicongel): Mittlere Zahl der Rezidive in 6 Monaten pro Versuchsperson: 2,4 ± 1,5 ; Mittlere Dauer: 5,5 ±1 Tage
b) Probengruppe (D20-Silicongel): Mittlere Zahl der Rezidive in 6 Monaten pro Versuchsperson: 2,2 ± 1,5 ; Mittlere Dauer: 2,0 ±1 Tage

Die Ergebnisse zeigen eine klare Wirksamkeit des D20-Silicongels zur Verringerung der Dauer eines HSV-2 Rezidivs. Nebenwirkungen wurden von den Testpersonen nicht beobachtet.

### Beispiel 13: Wirksamkeit von D20 zur Warzentherapie von HPV-basierten Warzen im klinischen Versuch (fällt nicht unter den Schutzbereich der vorliegenden Erfindung)

Es wurden 10 gesunde Freiwillige im Alter von 21- 41 Jahren (5 weiblich, 5 männlich) für diese Studie ausgewählt. Alle ausgewählten Testpersonen hatten eine Historie von Warzen des Typs Verrucae Vulgares, die sich insbesondere an den Fingern manifestierten. Es wurden nur solche Testpersonen ausgewählt, die an mindestens zwei Fingern Warzen dieses Typs aufwiesen.

Bei allen Testpersonen wurde zunächst die Verhornungsschicht der Warzen an zwei Fingern durch lokale Vereisung mittels flüssigen Stickstoff entfernt. Am Tage darauf wurden die behandelten Warzen bezüglich ihres über die Haut herausstehenden Volumens durch eine berührungslose optische Methode (3D-Warzenvermessung mittels Streifenprojektion System ATOS-1 der Fa. GOM, Bibertal bei Ulm, Deutschland) vermessen und die Daten für jede Testperson und für jede einzelne Warze protokolliert.

Anschließend wurden die Testpersonen randomisiert in 2 Gruppen (Probengruppe und Kontrollgruppe) zu je 5 Personen eingeteilt. Bei der Probengruppe wurden an zwei zuvor in oben beschriebener Weise behandelten Fingern direkt auf den Warzen nach Beispiel 5 hergestellte, 1,5 mm dicke D20-Agarosegele (in Quadrate von 1 cm² Größe geschnitten, hergestellt aus reinem D20) aufgebracht und mittels eines darüber angebrachten Tegaderm-Pflasters (Typ 16004 von 3M Corp., minneapolis, USA) zugeschnitten auf Größe 5cm x 2cm) semi-okklusiv fixiert. Bei der Kontrollgruppe wurde unter ansonsten identischen Bedingungen ein analog nach Beispiel 5 hergestelltes H2O-Agarosegel auf den Warzen aufgebracht und fixiert. Bei beiden Gruppen wurden pro Testperson mindestens 3 Warzen auf die beschriebene Weise behandelt.

Nach fünf Tagen wurden Pflaster und Gele entfernt und die Warzenvolumina optisch vermessen. Aus dem Vergleich mit den vor Applikation der Gele bestimmten individuellen Warzen-Volumina wurde die prozentuale Volumenzunahme berechnet. Durch Mittelwertbildung wurde die mittlere prozentuale Volumenzunahme <%V_{w}> der Warzen der jeweiligen Gruppe berechnet.

Ergebnisse:
Kontrollgruppe <%V_{w}> = (190±20)%;
Probengruppe <%V_{w}>= (105±5)%.

Für die Probengruppe (D2O-Agarosegel) hatte das Warzenvolumen im Beobachtungszeitraum innerhalb des Meßfehlers nicht zugenommen.

### Tabelle 1:

Wirksamkeit von D20 bei HSV-1 infizierten Meerschweinchen (Beispiel 10). Dargestellt ist die mittlere Zahl und Größe von Läsionen sowie die mittlere Zahl von HSV-1 pro Keratinozytenzelle (jeweils bezogen auf die mit Gel behandelte Rückenfläche) 5 Tage nach der Infektion mit HSV-1. Unmittelbar nach Infektion wurden die 4 Gruppen von je 3 Tieren wie folgt behandelt: Probengruppe 1 erhielt alle 3 Stunden ein nach Beispiel 1 mit 100% D20 hergestelltes D20-Carbopolgel auf dem Rücken aufgebracht (ca. 1,0 Gramm Gel pro Applikation). Die Kontrollgruppe 1 erhielt eine gleiche Menge eines nach Beispiel 1 hergestellten H2O-Carbopolgel (aus 100% H2O) zu identischen Zeitpunkten aufgetragen. Probengruppe 2 erhielt ausschließlich Trinkwasser, welchem 35% D20 beigemischt war und keine topische Applikation eines Gels. Die Kontrollgruppe 2 wurde nach der Infektion keinerlei weiterer Behandlung unterzogen.

| | Mittl. Läsionenzahl | Mittl. Größe der Läsionen (mm²) | Mittl. Zahl von HSV1 Viren pro Zelle |
|---|---|---|---|
| Probengruppe 1 (D2O-Carbopolgel) | 2 | 16±3 | 6±3 |
| Probengruppe 2 (D20 im Trinkwasser) | 3 | 19±3 | 11±4 |
| Kontrollgruppe 1 (H20- Carbopolgel) | 9 | 25±3 | 24±4 |
| Kontrollgruppe 2 | 10 | 23±3 | 26±4 |

### Tabelle 2:

Gesamtzahl direkter und indirekter Läsionen an insgesamt 32 Testpersonen im Zeitraum bis 15 Tage nach der UV- Bestrahlung (Beispiel 11). Laut obiger Angaben (Beispiel 11) treten direkte Läsionen im Zeitraum bis zu 2 Tage nach der Bestrahlung auf, indirekte Läsionen im Zeitraum 2 bis 15 Tage. Unmittelbar nach der Bestrahlung und von diesem Zeitpunkt an alle 2,5 Stunden (außer nachts) wurde der bestrahlte Lippenbereich bei den jeweils 8 Testpersonen der vier Versuchsgruppen mit D20- Carbopolgel (hergestellt nach Beispiel 1 mit 100% D20), mit H2O-Carbopolgel (Placebo, hergestellt nach Beispiel 1 mit 100% H2O), mit Zovirax Creme (Aciclovir im Cremespender von GlaxoSmithKline) oder mit einer nach Beispiel 7 hergestellten D20-Creme bestrichen, in welche zusätzlich 20 Gew% Zovirax Creme homogen eingemischt war, bestrichen.

| **Gel / Creme** | **Indirekte Läsionen (Gesamtzahl)** | **Direkte Läsionen (Gesamtzahl)** |
|---|---|---|
| D2O-Carbopolgel | 2 | 1 |
| H20-Carbopolgel | 6 | 8 |
| Zovirax Creme | 5 | 5 |
| D2O/Zovirax Creme | 2 | 1 |

## Patentansprüche

1. Deuteriumoxid zur Verwendung für die Prophylaxe und/oder Therapie von Herpesvirus-basierten Erkrankungen der Haut, wobei Deuteriumoxid rein, oder in Lösung mit Wasser als Lösungsmittel in einer Konzentration von 1 % bis 98% bezogen auf den Gesamtwassergehalt der Lösung, vorliegt.

2. Deuteriumoxid zur Verwendung nach Anspruch 1, wobei die Herpesvirus-basierten Erkrankungen der Haut durch Herpes simplex Virus Typ 1 oder Herpes simplex Virus Typ 2 hervorgerufen werden.

3. Deuteriumoxid zur Verwendung nach Anspruch 1 oder 2, wobei es sich um Herpesvirus-basierte Erkrankungen der Haut, ausgewählt aus der Gruppe bestehend aus Herpes labiales, Herpes nasalis, Keratoconjunctivitis herpetica, Stomatitis herpetica, Herpes facialis, Herpes buccalis, Herpes genitales, Herpes perianalis und Herpes glutealis und Herpes zoster, handelt.

4. Deuteriumoxid zur Verwendung nach einem der vorangehenden Ansprüche, wobei das Deuteriumoxid topisch auf die Haut appliziert wird.

5. Deuteriumoxid zur Verwendung nach einem der vorangehenden Ansprüche, wobei das D20 in Kombination mit mindestens einem weiteren pharmazeutischen Wirkstoff und/oder mindestens einem weiteren nicht-pharmazeutischen Wirkstoff verwendet wird.

6. Deuteriumoxid zur Verwendung nach Anspruch 5, wobei der mindestens eine weitere pharmazeutische Wirkstoff ausgewählt ist aus der Gruppe, bestehend aus Virusstatika, Proteinen, Peptiden, Nukleinsäuren und immunosuppressiven Wirkstoffen.

7. Deuteriumoxid zur Verwendung nach Anspruch 5, wobei der mindestens eine weitere nicht-pharmazeutische Wirkstoff ausgewählt ist aus der Gruppe, bestehend aus pharmazeutisch verträglichen anorganischen oder organischen Säuren oder Basen, Polymeren, Copolymeren, Block-Copolymeren, Einfachzucker, Mehrfachzucker, ionische und nicht-ionische Tenside oder Lipiden sowie Mischungen hiervon, Albumin, Transferrin und DNA-Reparatur-Proteinen, wie Kinase-Inhibitoren.

8. Deuteriumoxid zur Verwendung nach einem beliebigen der vorangegangenen Ansprüche, wobei das D20 mit einem Pflaster oder einer Bandage topisch appliziert wird, und wobei das Pflaster oder die Bandage vorzugsweise in Kombination mit mindestens einer Membran oder mindestens einer Folie verwendet wird.

9. Deuteriumoxid zur Verwendung nach Anspruch 8, wobei die Membran oder Folie eine mikro- oder nanoporöse Membran oder eine mikro- oder nanoporöse Folie ist.

10. Deuteriumoxid zur Verwendung nach einem der Ansprüche 1 bis 7, wobei das D20 als Aerosol topisch appliziert wird.

11. Deuteriumoxid zur Verwendung nach einem beliebigen der vorangegangenen Ansprüche, wobei D20 als Formulierung topisch appliziert wird, und wobei die Formulierung vorzugsweise eine Salbe, Creme, Gel oder Hydrogel ist.

## Claims

1. Deuterium oxide for use for the prevention and/or treatment of herpes virus-based diseases of the skin, wherein deuterium oxide is present in pure form or in solution with water as a solvent at a concentration of 1% to 98% in relation to the overall water content of the solution.

2. Deuterium oxide for use according to claim 1, wherein the herpes virus-based diseases of the skin is caused by herpes simplex virus type 1 or herpes simplex virus type 2.

3. Deuterium oxide for use according to claim 1 or 2, wherein the herpes virus-based diseases of the skin is selected from the group consisting of herpes labialis, herpes nasalis, keratoconjunctivitis herpetica, stomatitis herpetica, herpes facialis, herpes buccalis, herpes genitalis, herpes perianalis and herpes glutealis and herpes zoster.

4. Deuterium oxide for use according to any of the preceding claims, wherein the deuterium oxide is applied topically to the skin.

5. Deuterium oxide for use according to any of the preceding claims, wherein the D20 is used in combination with at least one further pharmaceutical active ingredient and/or at least one other non-pharmaceutical active ingredient.

6. Deuterium oxide for use according to claim 5, wherein the at least one further pharmaceutical active ingredient is selected from the group consisting of virustatics, proteins, peptides, nucleic acids and immunosuppressant active ingredients.

7. Deuterium oxide for use according to claim 5, wherein the at least one further non-pharmaceutical active ingredient is selected from the group consisting of pharmaceutically compatible inorganic or organic acids or bases, polymers, copolymers, block copolymers, monosaccharides, polysaccharides, ionic and non-ionic surfactants or lipids and mixtures thereof, albumin, transferrin and DNA repair proteins such as kinase inhibitors.

8. Deuterium oxide for use according to any of the preceding claims, wherein the D20 is administered topically with a patch or a bandage, and wherein the patch or the bandage preferably is used in combination with at least one membrane or at least one film.

9. Deuterium oxide for use according to claim 8, wherein the membrane or film is a micro- or nanoporous membrane or a micro- or nanoporous film.

10. Deuterium oxide for use according to any of claims 1 to 7, wherein the D20 is administered topically as an aerosol.

11. Deuterium oxide for use according to any of the preceding claims, wherein D20 is administered topically as a formulation, and wherein the formulation preferably is an ointment, a cream, a gel or a hydrogel.

## Revendications

1. Oxyde de deutérium pour son utilisation pour la prophylaxie et/ou la thérapie de maladies de la peau provenant d'un virus de l'herpès, dans lequel l'oxyde de deutérium se présente sous forme pure ou en solution avec de l'eau comme solvant à une concentration de 1 % à 98 % par rapport à la teneur totale en eau de la solution.

2. Oxyde de deutérium pour son utilisation selon la revendication 1, dans lequel les maladies de la peau provenant d'un virus de l'herpès ont été provoquées par le virus de l'herpès simplex de type 1 ou le virus de l'herpès simplex de type 2.

3. Oxyde de deutérium pour son utilisation selon la revendication 1 ou 2, dans lequel les maladies de la peau provenant d'un virus de l'herpès sont choisies dans le groupe consistant en l'herpès labial, l'herpès nasal, la kératoconjonctivite herpétique, la stomatite herpétique, l'herpès facial, l'herpès buccal, l'herpès génital, l'herpès périanal et l'herpès glutealis et l'herpes zoster.

4. Oxyde de deutérium pour son utilisation selon l'une des revendications précédentes, dans lequel l'oxyde de deutérium est administré par voie topique sur la peau.

5. Oxyde de deutérium pour son utilisation selon l'une des revendications précédentes, dans lequel le D20 est utilisé en combinaison avec au moins une autre substance active pharmaceutique et/ou au moins une autre substance active non pharmaceutique.

6. Oxyde de deutérium pour son utilisation selon la revendication 5, dans lequel l'au moins une autre substance active pharmaceutique est choisie dans le groupe consistant en les antiviraux, les protéines, les peptides, les acides nucléiques et les substances actives immunosuppressives.

7. Oxyde de deutérium pour son utilisation selon la revendication 5, dans lequel l'au moins une autre substance active non pharmaceutique est choisie dans le groupe consistant en les acides ou les bases inorganiques ou organiques pharmaceutiquement acceptables, les polymères, les copolymères, les copolymères blocs, les monosaccharides, les polysaccharides, les tensioactifs ioniques et non ioniques ou les lipides et des mélanges de ceux-ci, l'albumine, la transferrine et les protéines de réparation de l'ADN comme les inhibiteurs des protéines kinases.

8. Oxyde de deutérium pour son utilisation selon l'une quelconque des revendications précédentes, dans lequel le D20 est administré topiquement avec un pansement ou un bandage et dans lequel le pansement ou le bandage est utilisé de préférence en combinaison avec au moins une membrane ou au moins un film.

9. Oxyde de deutérium pour son utilisation selon la revendication 8, dans lequel la membrane ou le film est une membrane micro- ou nanoporeuse ou un film micro- ou nanoporeux.

10. Oxyde de deutérium pour son utilisation selon l'une des revendications 1 à 7, dans lequel le D20 est administré topiquement comme aérosol.

11. Oxyde de deutérium pour son utilisation selon l'une quelconque des revendications précédentes, dans lequel le D20 est appliqué topiquement en tant que formulation et dans lequel la formulation est de préférence une pommade, une crème, un gel ou un hydrogel.
